# EUROPEAN PATENT SPECIFICATION

(11) **EP 2 445 530 B1**
(45) Date of publication and mention of the grant of the patent: **07.09.2016**
(21) Application number: 10792463.1
(22) Date of filing: 24.06.2010
(51) Int. Cl.: A61K 39/395, C07K 16/30, G01N 33/574

(54) **METHOD FOR TREATING CHRONIC LYMPHOCYTIC LEUKEMIA**
VERFAHREN ZUR BEHANDLUNG CHRONISCHER LYMPHOZYTENLEUKÄMIE
PROCÉDÉ POUR TRAITER LA LEUCÉMIE LYMPHOCYTAIRE CHRONIQUE

(30) Priority: 24.06.2009 US 269398 P
(43) Date of publication of application: 02.05.2012
(73) Proprietor: The Feinstein Institute for Medical Research, Manhasset, NY 11030 (US)
(72) Inventor: CALISSANO, Carlo, I-20127 Milano (IT); CHIORAZZI, Nicholas, Tenafly, NJ 07670 (US); DAMLE, Rajendra, N., Lynbrook, NY 11563 (US); PATTEN, Piers, Port Washington, NY 11050 (US)
(74) Representative: Paemen, Liesbet R.J.
(86) International application number: PCT/US2010/001840
(87) International publication number: WO 2010/151341

(56) References cited:
- WO-A1-00/44788
- US-A1- 2007 202 534
- US-A1- 2008 219 971
- TAHAMTAN AHMADI ET AL: "Chronic lymphocytic leukemia: new concepts and emerging therapies", CURRENT TREATMENT OPTIONS IN ONCOLOGY, CURRENT SCIENCE, PHILADELPHIA, PA, US, vol. 10, no. 1-2, 1 April 2009 (2009-04-01), pages 16-32, XP002665079, ISSN: 1534-6277, DOI: 10.1007/S11864-008-0079-8
- ANTONELLA ZUCCHETTO ET AL: "Signature of B-CLL with different prognosis by Shrunken centroids of surface antigen expression profiling", JOURNAL OF CELLULAR PHYSIOLOGY, vol. 204, no. 1, 1 July 2005 (2005-07-01), pages 113-123, XP055059427, ISSN: 0021-9541, DOI: 10.1002/jcp.20269
- R. N. DAMLE ET AL: "CD38 expression labels an activated subset within chronic lymphocytic leukemia clones enriched in proliferating B cells", BLOOD, vol. 110, no. 9, 1 November 2007 (2007-11-01), pages 3352-3359, XP055059433, ISSN: 0006-4971, DOI: 10.1182/blood-2007-04-083832
- CARLO CALISSANO ET AL: "Intraclonal complexity in chronic lymphocytic leukemia: fractions enriched in recently born/divided and older/quiescent cells.", MOLECULAR MEDICINE, vol. 17, no. 11-12, 1 January 2011 (2011-01-01), pages 1-1382, XP055059435, ISSN: 1076-1551, DOI: 10.2119/molmed.2011.00360
- BOGNER ET AL.: 'Cycling B-CLL cells are highly susceptible to inhibition of the proteasome: Involvement of p27, early D-type cyclins, Bax, and caspase-dependent and -independent pathways.' EXPERIMENTAL HEMATOLOGY vol. 31, 2003, pages 218 - 225, XP008149167
- S. Deaglio: "In-tandem insight from basic science combined with clinical research: CD38 as both marker and key component of the pathogenetic network underlying chronic lymphocytic leukemia", Blood, vol. 108, no. 4, 15 August 2006 (2006-08-15), pages 1135-1144, XP055102541, ISSN: 0006-4971, DOI: 10.1182/blood-2006-01-013003
- Dilip Gupta ET AL: "Small Lymphocytic Lymphoma with Perifollicular, Marginal Zone, or Interfollicular Distribution", Modern Pathology, vol. 13, no. 11, 1 November 2000 (2000-11-01), pages 1161-1166, XP055102548, ISSN: 0893-3952, DOI: 10.1038/modpathol.3880214
- VAN BOCKSTAELE F ET AL: "Prognostic markers in chronic lymphocytic leukemia: A comprehensive review", BLOOD REVIEWS, CHURCHILL LIVINGSTONE, AMSTERDAM, NL, vol. 23, no. 1, 1 January 2009 (2009-01-01), pages 25-47, XP025878928, ISSN: 0268-960X, DOI: 10.1016/J.BLRE.2008.05.003 [retrieved on 2008-07-02]
- Vladimir Jurisic ET AL: "Analysis of CD23 antigen expression in B-chronic lymphocytic leukaemia and its correlation with clinical parameters", Medical Oncology, vol. 25, no. 3, 9 January 2008 (2008-01-09), pages 315-322, XP055151818, ISSN: 1357-0560, DOI: 10.1007/s12032-007-9038-7
- Antonella Zucchetto ET AL: "A scoring system based on the expression of six surface molecules allows the identification of three prognostic risk groups in B-cell chronic lymphocytic leukemia", Journal of Cellular Physiology, vol. 207, no. 2, 1 May 2006 (2006-05-01), pages 354-363, XP055059430, ISSN: 0021-9541, DOI: 10.1002/jcp.20570
- Michael Stanglmaier ET AL: "Bi20 (fBTA05), a novel trifunctional bispecific antibody (anti-CD20 x anti-CD3), mediates efficient killing of B-cell lymphoma cells even with very low CD20 expression levels", International Journal of Cancer, vol. 123, no. 5, 1 September 2008 (2008-09-01), pages 1181-1189, XP055089407, ISSN: 0020-7136, DOI: 10.1002/ijc.23626

## Description

### Field of the Invention

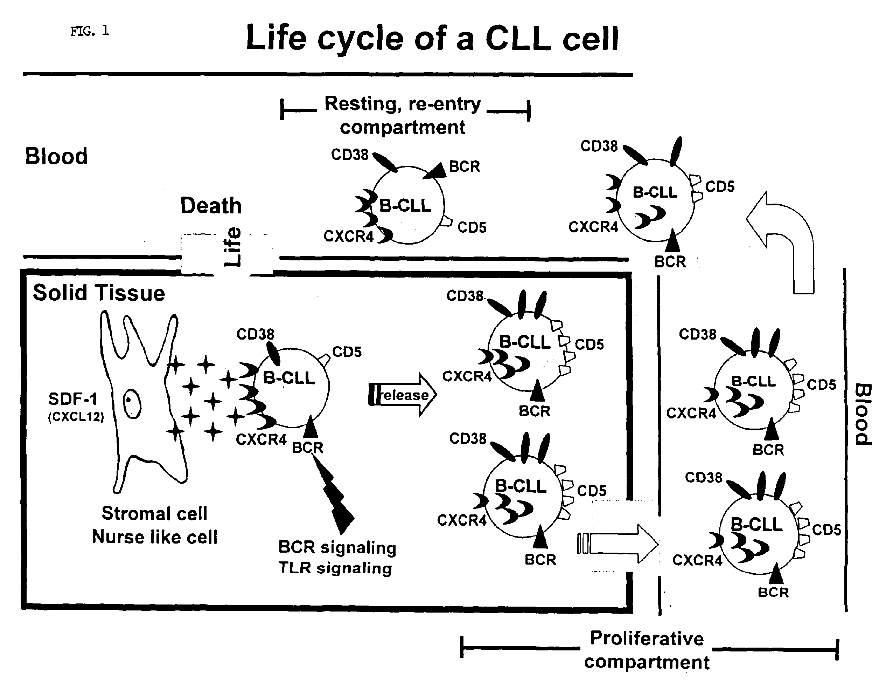

The present invention relates to the treatment of chronic lymphocytic leukemia using agents that preferentially target proliferative cells ("proliferative compartment") and also resting cells ("resting, re-entry compartment") within CLL clones (Figure 1).

### Background of the Invention

B-cell type chronic lymphocytic leukemia (CLL) is the most common leukemia in western countries. The clinical course of CLL patients varies; some patients are extremely stable and survive for 2-3 decades with no specific treatments, whereas others experience a much more aggressive course which is fatal regardless of therapeutic attempts. In both types of patients, CLL is incurable (1).

CLL cells are defined by the surface membrane co-expression of B-cell specific molecules (e.g., CD19 and CD20) and CD5 (1). The B-cell specific antigen CD20 is expressed by normal B lymphocytes throughout maturation to the plasma cell stage. Albeit expressed at a low density on CLL cells (2) the anti-CD20 mAb, Rituximab, in combination with fludarabine, exhibits considerable efficacy in CLL (3). Furthermore, Rituximab in combination with high-dose methylprednisolone is efficacious in fludarabine-refractory CLL (4).

Activated B cells express other surface molecules including CD23. CD23 (the low affinity receptor for IgE; Fc epsilon RII) is a 45kDa membrane glycoprotein primarily expressed on the surface of activated normal human B cells and on many CLL cells (5). Lumiliximab is a chimeric macaque-human monoclonal antibody (mAb) to CD23 that eliminates CLL cells and CD23-expressing B cells by apoptosis; apoptosis is accompanied by down-regulation of anti-apoptotic proteins such as Bcl-2, Bcl-X(L), and XIAP, activation of pro-apoptotic molecules like Bax, and release of cytochrome c from mitochondria. Addition of lumiliximab to rituximab or fludarabine results in synergistic cytotoxicity of primary CLL cells and CD23-expressing B-cell lines (6).

Since CD38 expression segregates CLL cases into clinically distinct subgroups (7), specific emphasis has been placed on understanding its role in CLL. CD38 is a surface membrane bound ectoenzyme that also functions as a receptor and signaling molecule. Expression of CD38 on normal B cells depends on the stage of cell maturation and can be induced upon cellular activation (8). Recent studies have connected CD38's prognostic value to its capacity to promote leukemic cell survival by interacting with stromal cells (9). In addition, within each CLL clone, the CD38- expressing fraction is enriched in cells expressing Ki-67 (10), a marker of proliferation (11), suggesting that CD38+ cells contain a cycling subset of CLL cells. Indeed, recent *in vivo* studies identified a small "proliferative compartment" of cells among the clonal population in CLL (12).

CD52 is expressed by most human mononuclear cells and by a variety of lymphoid neoplasms, including CLL. Alemtuzumab (Campath-1H), a humanized IgG1 monoclonal antibody, is an anti-CD52 mAb approved for single agent use in fludarabine-refractory CLL patients. In heavily pretreated patients with CLL, the overall response rate (ORR) is ~35%, and in previously untreated patients, the ORR is >80% (13). Adverse events associated with alemtuzumab administration include myelosuppression as well as profound cellular immune dysfunction with the associated risk of viral reactivation and opportunistic infections. Studies detailing the mechanism of action of alemtuzumab as well as new strategies for prevention of opportunistic infections are in progress (13).

In addition to expressing these surface markers, CLL cells express, at varying surface densities, CXCR4, the receptor for SDF-1/CXCL12, a chemokine produced by stromal cells. CXCR4 is also expressed by normal B and T lymphocytes and plays a role in cell migration and homoeostasis. CLL cells interact via CXCR4 with cells and chemokines of the microenvironment, thereby promoting their survival and growth (14,15). Furthermore, determining expression levels of CXCR4 has prognostic value (16).

Finally, integrins expressed on CLL cells have important functions for the leukemic cells as well as providing prognostic relevance. CD49d is an example of this, permitting CLL cells to transit to and from solid tissue compartments (17,18). CD62L, also known as L-selectin, is a homing receptor that allows CLL cells to enter secondary lymphoid tissues via high endothelial venules. CD27 is a member of the tumor necrosis factor receptor superfamily that is upregulated on activated lymphoid cells. It binds CD70, and plays a key role in regulating B-cell activation by transducing signals leading to the activation of NF-κB and MAPK8/JNK. CD48 is an activation-associated cell surface molecule expressed primarily on stimulated human lymphocytes. It is the ligand for CD2 on T lymphocytes and therefore may regulate the interaction between activated T and B cells. CD 100 is a semiphorin that induces B-cell aggregation and supports B cell survival and growth.

Over the past decade, considerable progress has been made in treating CLL. In particular, combination immune and chemotherapies have been effective in eliminating most members of the CLL clone from the blood of many CLL patients and from the solid lymphoid tissues (bone marrow, lymph nodes, and spleen) in some patients. In particular, combining fludarabine with cyclophosphamide (19) and/or anti-CD20 mAb (Rituxan) (20) in a series of administrations (as many as 6) has been especially effective. Nevertheless, all patients inevitably relapse. Thus, more effective regimens are needed.

In addition, "consolidation regimens" are being added to these chemo-immunotherapeutic approaches. Anti-CD52 (Alemtuzumab) can be effective in this setting (21). Unfortunately, because of their frequent lack of specificity for CLL cells, these consolidating regimens, in particular Alemtuzumab, have had major adverse side effects, especially severe cytopenias and opportunistic infections due to the further assault on an immune system already compromised by the inductive regimens.

Thus there remains a major need for more effective inductive regimens, especially those targeting specifically the leukemic cells and their interactions with the microenvironment that do not have significant "off target" actions and toxicities. Similarly, consolidation regimens with the same level of specificity and lack of toxicity are essential.

Tahamtan et al (Current treatment options in oncology, current science, Philadalphia, PA, US 2009. 10:16-32) discloses the use of separate monoclonal antibodies in the treatment of CLL.

WO 00/44788 (Idec Pharma Corp, US 2000) discloses heterodimers comprising a CD20 and a CD23 binding part.

Deaglio et al. (Blood 2006. 108:1135-1144) describes CD38 as both marker and key component of the pathological network underlying chronic lymphocytic leukemia. Deaglio et al. discloses that CD5+ and CD23+ B-cell phenotype establishes correct diagnosis of CLL.

Gupta et al (Modern Pathology 2000. 13:1161-1166) studies small lymphocytic lymphoma with perifollocular, maginal zone or interfollicular distribution. Gupta et al. discloses several studies indicating that the CD38 marker has prognostic value in CLL.

Van Bockstaele et al. (Blood Reviews, Churchill Livingstone, Amsterdam, NL 2009. 23:25-47) provides a review on prognostic markers in chronic lymphocytic leukemia. Van Bockstaele et al. discloses that CD38 is a negative prognostic marker and a diagnostic marker.

Jurisic et al (Medical Oncology 2008. 25:315-322) and D7 Damle et al (Blood 2007. 110:3352-3359) are directed to methods of diagnosing CLL by detecting CD expression on cells; CD23 and CD38 antigen expression in CLL are studied, respectively.

Stanglmaier et al. (Internation Journal of Cancer 2008. 123: 1181-1189) discloses the use of CD20/CD3 bispecific antibodies in the treatment of B-CLL.

Zucchetto et al (Journal of Cellular Physiology 2006. 207:354-363) describes a scoring system based on the expression of surface molecules for the identifiation of B-CLL.

### Summary of the Invention

The present specification describes a method for treating a subject having chronic lymphocytic leukemia (CLL) comprising administering to the subject one or more agents that target cell surface membrane antigens expressed preferentially on cells of the proliferative compartment, or on the resting, re-entry compartment, of a CLL clone of the subject to treat chronic lymphocytic leukemia in the subject. In the preferred embodiment described herein, the agent or agents treat the subject by reducing or depleting the proliferating subpopulation and/or the resting, re-entry subpopulation of a CLL clone of the subject. Accordingly, described herein is a method of depleting a CLL clone in a subject with CLL comprising introducing an agent that depletes a proliferating subpopulation, and/or a resting, re-entry subpopulation, of the CLL clone of the subject.In accordance with the present invention, the cell surface membrane antigens expressed preferentially on cells of the proliferative CLL compartment or the resting, re-entry compartment of a CLL clone of the subject may be identified by associating members of a set of surface membrane antigens with the cells in the subject's CLL leukemic clone that divide most vigorously. In the preferred embodiment of the methods described herein, the cell surface membrane antigens are selected from the set comprising one or more of any combination of CD19, CD20, CD5, CXCR4, CD11a, CD23, CD27, CD38, CD48, CD49d, CD52, CD62L, and CD100. In a more preferred embodiment described herein, the differential expression by cells of the proliferative compartment comprises down regulated expression of CXCR4 and up-regulate expression of one or more of any combination of CD5, CD11a, CD20, CD23, CD27, CD38, CD48, CD49d, CD52, CD62L, and CD100. In other embodiments described herein, the proliferating cells are identified by enrichment in ²H-labeled DNA or Ki-67 expression, and preferably by both enrichment in ²H-labeled DNA and Ki-67 expression.

Alternatively, the cell surface membrane antigens expressed preferentially on cells of the resting, re-entry compartment of a CLL clone of the subject may be identified by associating members of a set of surface membrane antigens with the cells in the subject's CLL leukemic clone that are attempting to re-enter a lymphoid tissue niche from the blood. In a preferred embodiment of the methods described herein, the cell surface membrane antigens are selected from the set comprising one or more of any combination of CD19, CD20, CXCR4, and CCR7. In a more preferred embodiment of the methods described herein, the differential expression by cells of the resting, re-entry compartment comprises down regulated expression of CD5, CD11a, CD20, CD23, CD27, CD38, CD48, CD49d, CD52, CD62L, and CD100 and up-regulated expression of one or more of any combination of CXCR4 and CCR7. In other embodiments of the methods described herein, the resting re-entry cells are identified by a paucity (relative to the fraction of labeled cells of the proliferative compartment) of ²H-labeled DNA and Ki-67 expression, and preferably by a paucity in both ²H-labeled DNA and Ki-67 expression.

In accordance with the method described herein, the subject is administered one or more agents that bind to at least two of any combination of a B cell-specific marker (such as CD 19, CD20 or CD23) with any of the following cell surface markers: CD11a, CD23, CD27, CD38, CD48, CD49d, CD52, CD62L, and CD100. The invention relates to an agent which is a bispecific antibody and which targets cell surface membrane antigens expressed preferentially on cells of the proliferative compartment of a chronic lymphocytic leukemia (CLL) clone wherein the agent can bind to CD19 and CD11a, CD19 and CD23, CD19 and CD27, CD19 and CD38, CD19 and CD48, CD19 and CD49d, CD19 and CD52, CD19 and CD62L, and CD 19 and CD100; CD20 and CD 11a, CD20 and CD23, CD20 and CD27, CD20 and CD38, CD20 and CD48, CD20 and CD49d, CD20 and CD52, CD20 and CD62L, and CD20 and CD100; CD23 and CD11a, CD23 and CD27, CD23 and CD38, CD23 and CD48, CD23 and CD49d, CD23 and CD52, CD23 and CD62L, and CD23 and CD100.

Preferably, the agent is a bi-specific antibody that binds to (i) CD19 and CD11a, (ii) CD19 and CD23, (iii) CD19 and CD27, (iv) CD19 and CD38, (v) CD19 and CD48, (vi) CD19 and CD49d, (vii) CD19 and CD52, (viii) CD19 and CD62L, and (ix) CD19 and CD100; (x) CD20 and CD11a, (xi) CD20 and CD23, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD23 and CD52, (xxv) CD23 and CD62L, and (xxvi) CD23 and CD100. Accordingly, the present invention provides an agent which is a bispecific antibody that binds to (i) CD23 and CD52, (ii) CD19 and CD23, (iii) CD19 and CD27, (iv) CD19 and CD38, (v) CD19 and CD48, (vi) CD19 and CD49d, (vii) CD19 and CD52, (viii) CD19 and CD62L, and (ix) CD19 and CD100; (x) CD20 and CD11a, (xi) CD 19 and CD11a, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD23 and CD62L, or (xxv) CD23 and CD100. In particular embodiments, the agent is a bispecific antibody that binds to CD23 and CD52. In a particular embodiment, the agent is a bispecific antibody that binds to CD20 and CD52

Alternatively, more than one agent (e.g., two, three, four or more agents) can be administered to treat CLL where each agent (e.g., antibody) binds to a different cell surface membrane antigen. In this regard, and by way of example, if two agents are antibodies, then two antibodies that preferably bind to (i) CD 19 and CD11a, respectively, (ii) CD 19 and CD23, respectively, (iii) CD19 and CD27, respectively, (iv) CD19 and CD38, respectively, (v) CD 19 and CD48, respectively, (vi) CD19 and CD49d, respectively, (vii) CD19 and CD52, respectively, (viii) CD19 and CD62L, respectively, and (ix) CD19 and CD100, respectively; (x) CD20 and CD11a, respectively, (xi) CD20 and CD23, respectively, (xii) CD20 and CD27, respectively, (xiii) CD20 and CD38, respectively, (xiv) CD20 and CD48, respectively, (xv) CD20 and CD49d, respectively, (xvi) CD20 and CD52, respectively, (xvii) CD20 and CD62L, respectively, and (xviii) CD20 and CD100, respectively; (xix) CD23 and CD11a, respectively, (xx) CD23 and CD27, respectively, (xxi) CD23 and CD38, respectively, (xxii) CD23 and CD48, respectively, (xxiii) CD23 and CD49d, respectively, (xxiv) CD23 and CD52, respectively, (xxv) CD23 and CD62L, respectively, and (xxvi) CD23 and CD100, respectively, can be administered to treat the CLL in the subject. It is within the confines of the present invention that the antibodies can be polyclonal or monoclonal antibodies. In addition, the antibodies for use in the present invention can be rodent (e.g., mouse), chimeric, humanized or human antibodies, and are preferably human antibodies. In addition, when more than one agent is used for therapy, the agents may be administered simultaneously or in tandem. It is also within the confines of the present invention that the agent or agents may be conjugated with or attached or bound to a toxin or radioligand. The agent or agents also may be administered in a manner that permit the agent or agents (alone or bound to a toxin or radioligand) to contact the cells of the proliferative compartment in the bone marrow or in the periphery to destroy or kill these cells, and to thereby treat CLL. In this regard, it is contemplated that the agent or agents (alone or bound to a toxin or radioligand) can be administered directly to the blood stream of the patient (e.g, by injection), directly to the bone marrow of the patient, or in conjunction with a bone marrow transplant (e.g., autologous or allogenic bone marrow transplant) in which the agents are present in the bone marrow transplant composition. Still further, it is within the confines of the present invention that the agent or agents can be administered prior to, following, or in combination with other therapeutic treatments for chronic lymphocytic leukemia, such as those described above. The agent may be an antibody, apatmer, peptide, or T lymphocyte or natural killer cell with a chimeric bispecific receptor reactive with a B-cell specific antigen (e.g., CD19 or CD20 or CD23) and any of the following: CD11a, CD23, CD27, CD38, CD48, CD49d, CD52, CD62L, and CD100), and is preferably an antibody, and most preferably, a bi-specific antibody.

Described herein is an agent that binds to at least two cell surface membrane antigens selected from the set of two or more of any combination of CD19, CD20, CD5, CXCR4, CD11a, CD23, CD27, CD38, CD48, CD49d, CD52, CD62L, and CD100. In the preferred embodiment described herein, the agent binds to at least two of any combination of CD19, CD20, CD5, CXCR4, CD11a, CD23, CD27, CD38, CD48, CD49d, CD52, CD62L, and CD100. According to the present invention an agent is provided which is a bispecific antibody and which targets cell surface membrane antigens expressed preferentially on cells of the proliferative compartment of a chronic lymphocytic leukemia (CLL) clone wherein the agent binds, and more preferably binds to (i) CD19 and CD11a, (ii) CD19 and CD23, (iii) CD19 and CD27, (iv) CD19 and CD38, (v) CD19 and CD48, (vi) CD19 and CD49d, (vii) CD 19 and CD52, (viii) CD 19 and CD62L, and (ix) CD 19 and CD100; (x) CD20 and CD11a, (xi) CD20 and CD23, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD23 and CD52, (xxv) CD23 and CD62L, and (xxvi) CD23 and CD100.

Accordingly, the present invention provides an agent which is a bispecific antibody and which targets cell surface membrane antigens expressed preferentially on cells of the proliferative compartment of a chronic lymphocytic leukemia (CLL) clone wherein the agent binds to (i) CD23 and CD52, (ii) CD19 and CD23, (iii) CD19 and CD27, (iv) CD19 and CD38, (v) CD19 and CD48, (vi) CD19 and CD49d, (vii) CD19 and CD52, (viii) CD19 and CD62L, and (ix) CD19 and CD100; (x) CD20 and CD11a, (xi) CD20 and CD23, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD 19 and CD11a, (xxv) CD23 and CD62L, or (xxvi) CD23 and CD100, for use in a method of treating CLL in an inductive regimen. In a particular embodiments, the agent is a bispecific antibody and which targets cell surface membrane antigens expressed preferentially on cells of the proliferative compartment of a chronic lymphocytic leukemia (CLL) clone wherein the agent binds to CD23 and CD52. In a particular embodiment, the agent is a bispecific antibody and which targets cell surface membrane antigens expressed preferentially on cells of the proliferative compartment of a chronic lymphocytic leukemia (CLL) clone wherein the agent binds to CD20 and CD52.

Also described herein is a combination or cocktail of agents, each of which binds to two or more of two cell surface membrane antigens selected from the set of any combination of CD19, CD20, CD11a, CD23, CD27, CD38, CD48, CD49d, CD52, CD62L, and CD100. In the preferred embodiment described herein, the agents binds to at least two of any combination of CD19, CD20, CD11a, CD23, CD27, CD38, CD48, CD49d, CD52, CD62L, and CD100, and more preferably bind to (i) CD 19 and CD11a, respectively, (ii) CD 19 and CD23, respectively, (iii) CD19 and CD27, respectively, (iv) CD19 and CD38, respectively, (v) CD19 and CD48, respectively, (vi) CD19 and CD49d, respectively, (vii) CD19 and CD52, respectively, (viii) CD19 and CD62L, respectively, and (ix) CD19 and CD100, respectively; (x) CD20 and CD11a, respectively, (xi) CD20 and CD23, respectively, (xii) CD20 and CD27, respectively, (xiii) CD20 and CD38, respectively, (xiv) CD20 and CD48, respectively, (xv) CD20 and CD49d, respectively, (xvi) CD20 and CD52, respectively, (xvii) CD20 and CD62L, respectively, and (xviii) CD20 and CD100, respectively; (xix) CD23 and CD11a, respectively, (xx) CD23 and CD27, respectively, (xxi) CD23 and CD38, respectively, (xxii) CD23 and CD48, respectively, (xxiii) CD23 and CD49d, respectively, (xxiv) CD23 and CD52, respectively, (xxv) CD23 and CD62L, respectively, and (xxvi) CD23 and CD100, respectively. The agents may be antibodies, aptamers or peptides, or T lymphocyte or natural killer cell with a chimeric bispecific receptor reactive with a B-cell specific antigen (e.g., CD19 or CD20 or CD23) and any of the following: CD11a, CD23, CD27, CD38, CD48, CD49d, CD52, CD62L, and CD100), and are preferably antibodies. The present invention provides a pharmaceutical composition comprising the agent of the present invention as described above. In addition, the present invention provides a pharmaceutical composition that comprises the agent or combination of agents of the invention as described above, together with pharmaceutically acceptable carriers. The formulation of agents such as antibodies, aptamers or peptides, and or T lymphocyte or natural killer cell with a chimeric bispecific receptor is well known to the skilled artisan.

The present specification also describes a method for treating a subject having chronic lymphocytic leukemia comprising administering to the subject one or more agents that target cell surface membrane antigens expressed preferentially on cells of the "resting, re-entry compartment" to treat chronic lymphocytic leukemia in the subject. In this regard, the cell surface membrane antigens expressed preferentially on cells may be identified by co-expression of a series of surface membrane molecules. In a preferred embodiment described herein, the cell surface membrane antigens are selected from one or more of any combination of CD19, CD20, CXCR4, and CCR7. Cells of the "resting, re-entry compartment" down regulate expression of one or more of any combination of CD5, CD11a, CD23, CD27, CD38, CD48, CD49d, CD52, CD62L, and CD100 and up-regulate expression of one or more of any combination of CXCR4, and CCR7. The present specification also describes an agent that binds to at least two cell surface membrane antigens selected from the set of two or more of any combination of CD19, CD20, CXCR4, and CCR7. In the preferred embodiment described herein, the agent binds to at least two of any combination of CD19, CD20, CXCR4, and CCR7, and more preferably binds to (i) CD19 and CXCR4, (ii) CD19 and CCR7, (iii) CD20 and CXCR4, (iv) CD20 and CCR7, (v) CD23 and CXCR4, and (vi) CD23 and CCR7. The agent may be an antibody, apatmer, peptide, or T lymphocyte or natural killer cell with a chimeric bispecific antibody receptor reactive with CD 19 or CD20, and with CXCR4 or CCR7, and is preferably an antibody, and most preferably, a bi-specific antibodand is preferably an antibody, and most preferably, a bi-specific antibody.

The present specification further describes a method for diagnosing chronic lymphocytic leukemia or monitoring the progression of chronic lympocytic leukemia comprising (i) identifying and quantifying the number of proliferative cells or resting, re-entry cells associated with chronic lympocytic leukemia, and (ii) correlating the type and quantity of the cells to a control to diagnose chronic lymphocytic leukemia or monitor the progression of chronic lympocytic leukemia. In accordance with the method described in the present specification, the cells may be cells of the "proliferative compartment" and/or cells of the "resting re-entry compartment". With respect to the cells of the proliferative compartment, these may be identified by associating members of a set of surface membrane antigens to identify cells in a CLL leukemic clone that divide most vigorously. Such cell surface membrane antigens are selected from one or more of any combination of CD19, CD20, CD11a, CD23, CD27, CD38, CD48, CD49d, CD52, CD62L, and CD100. In the preferred embodiment described herein, the cells of the proliferative compartment down regulate expression of CXCR4 and CXCR7 and up-regulate expression of one or more of any combination of CD5, CD11a, CD23, CD27, CD38, CD48, CD49d, CD52, CD62L, and CD100. The proliferating cells also may be further characterized or identifed by enrichment in ²H-labeled DNA or Ki-67 expression, or by both enrichment in ²H-labeled DNA and Ki-67 expression. With respect to the cells of the "resting, re-entry compartment", these cells are preferably identified by cells that down regulate expression of one or more of any combination of CD5, CD11a, CD23, CD27, CD38, CD48, CD49d, CD52, CD62L, and CD100 and up-regulate expression of one or more of any combination of CXCR4 and CCR7.Additional objects of the specification will be apparent by the description which follows.

### Brief Description of the Figures

Figure 1. Life cycle of a CLL cell. Figure 1 is a cartoon schematically depicting the inventors' conception of the natural history or life cycle of prototypical CLL cells. CLL cells are generated or "born" in a proliferative compartment in lymphoid solid tissues (bone marrow, spleen, lymph node, and the like). As depicted in the brown box at the lower left of the figure, CLL cells are initially closely associated with normal cells such as stromal cells, "nurse-like" cells, T cells, and others (trophic niche) and communicate in the niche through the binding of both soluble (e.g., CXCL12 - CXCR4 interactions) and membrane-bound (e.g., (auto)antigen - B cell receptor("BCR") interactions) signaling molecules. Upon activation and/or dividing, CLL progeny separate and release from the trophic niche. As shown at the right-hand side of the brown box, these activated/dividing CLL cells down-regulate expression of CXCR4 and CCR7 and up-regulate expression of a number of surface membrane molecules (e.g., CD5, CD38, CD23, CD52, CD11a, CD49d, and others). Some recently divided cells exit the solid tissue compartment and enter the blood and lymphatic systems where they join the circulating bulk of the leukemic clone. As they traverse the blood and lymphatic circulation, the surface membrane phenotype of these cells changes. This involves down-regulation of a number of surface markers (e.g., CD5, CD38, CD23, CD52, CD11a, CD49d, and others) and up-regulation of others (e.g., CXCR4, CCR7 and others) leading to cells that represent a "resting" population of the CLL clone (depicted in upper portions of the Figure). Based on in vitro studies, cells of this compartment are fated to die unless they successfully encounter other cells that prevent apoptosis (e.g., stromal cells, nurse-like cells, T cells, and others) that would be present upon re-entering lymphoid solid tissues and populating trophic niches. Inventors also teach methods and reagents to treat CLL by targeting the extended surface membrane phenotype of this "resting, re-entry" compartment to preferentially eliminate this population or to prevent its successful re-entry to the trophic niche.

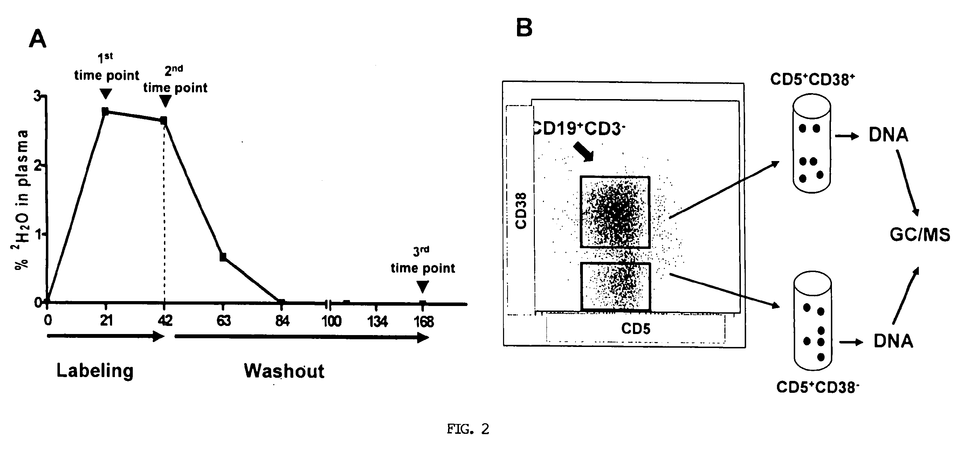

Figure 2A-2B. Procedure for determining ²H enrichments in CD38⁺ and CD38⁻ CLL cells and time point selection in relation to ²H availability in body water. A. Deuterium enrichment measured in plasma of body water compartment of a representative case (CLL875). CLL cells were flow-sorted at the 3 time points indicated by arrows. Vertical dashed line indicates time when ²H₂O intake was ended. B. After gating CD19⁺CD3⁻ cells, CD5⁺ cells were flow sorted based on their expression of CD38. ²H in genomic DNA was then measured by GC/MS.

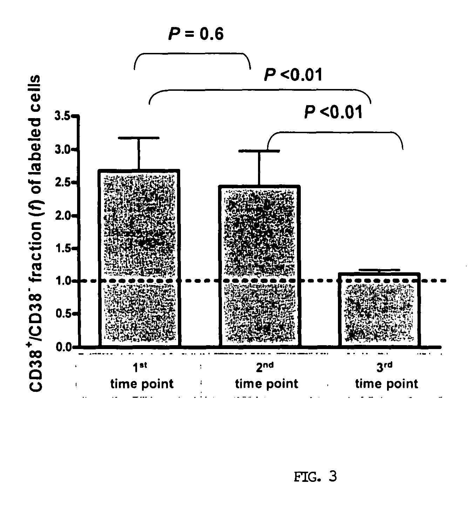

Figure 3. Ratios of ²H enrichment in CD38⁻ and CD38⁺ CLL cells during the course of the ²H₂O protocols. The ratio of CD38⁺/CD38⁻ fractions (*ƒ*) of labeled cells (Table 2) was averaged. Bars represent each of the 3 time points studied. Significant differences in ²H incorporation between the CD38⁻ and CD38⁺ CLL subpopulations were achieved early and maintained during labeling period, whereas they disappeared during washout.

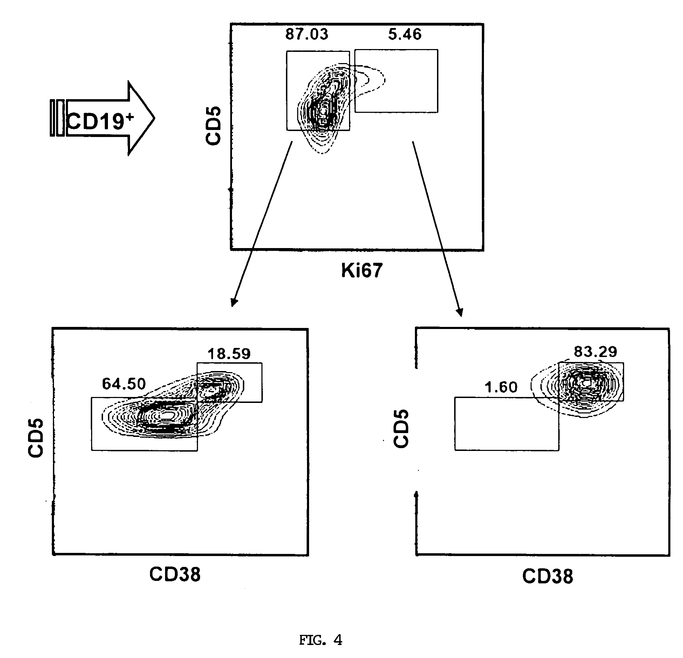

Figure 4. CD38 expression in Ki67⁺ and Ki67⁻ CLL cells. PBMCs from CLL822 were incubated with fluorochrome-labeled mAbs reactive with CD19, CD5, CD38 and, after cell permeabilization, with Ki67. Cells were first gated for CD19 and then analyzed as reported in the figure. Upper plot: CLL clone contains ~5% Ki67⁺ cells. Lower plots: Percent of CD38⁺ cells in the CD19⁺CD5⁺Ki67⁺ fraction (83%; lower right) is much higher compared to that observed in the CD19⁺CD5⁺Ki67⁻ fraction (18%; lower left).

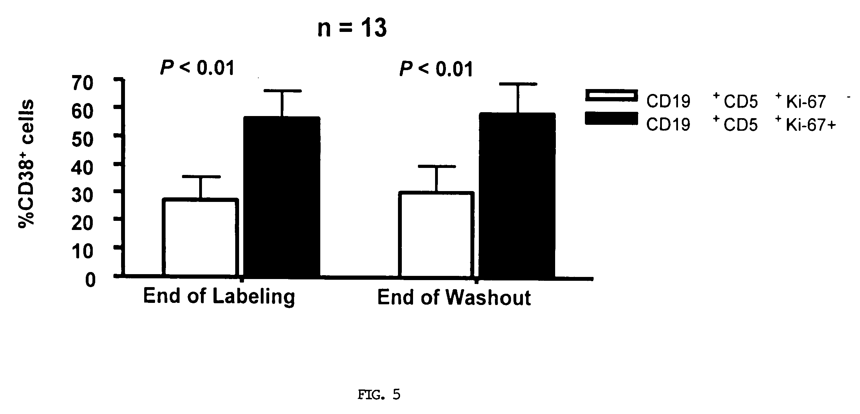

Figure 5. CD38 expressing cells are enriched in Ki67⁺ versus Ki67⁻ CLL cells. Data represent the composite of analyses for 13 CLL patients tested at the end of ²H₂0 labeling and at the end of washout. At both time points, Ki67⁺ fraction of CLL cells contains more CD38-expressing cells than the Ki67⁻ fraction.

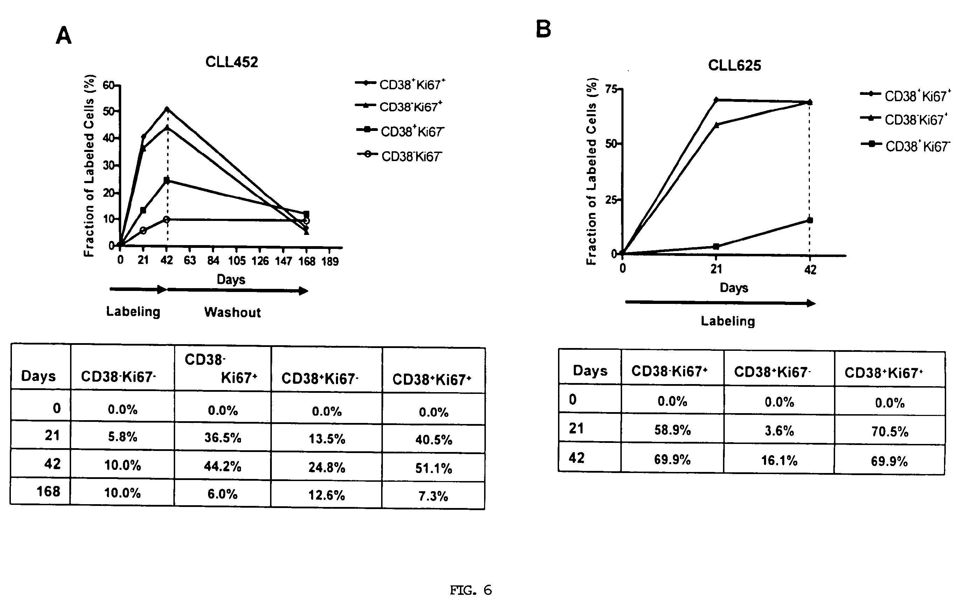

Figure 6A-6B. ²H enrichment in CLL clonal fractions sorted based on CD38 and Ki67. CLL cells from CLL452 (B) and CLL625 (C) were flow-sorted based on CD38 and Ki67 expression and ²H was measured in the sorted fractions. Curves represent the fraction (*ƒ*) of labeled cells reported in the tables below. Ki67⁺ cells, both CD38⁻ and CD38⁺, incorporated more ²H than their Ki67⁻ counterparts.

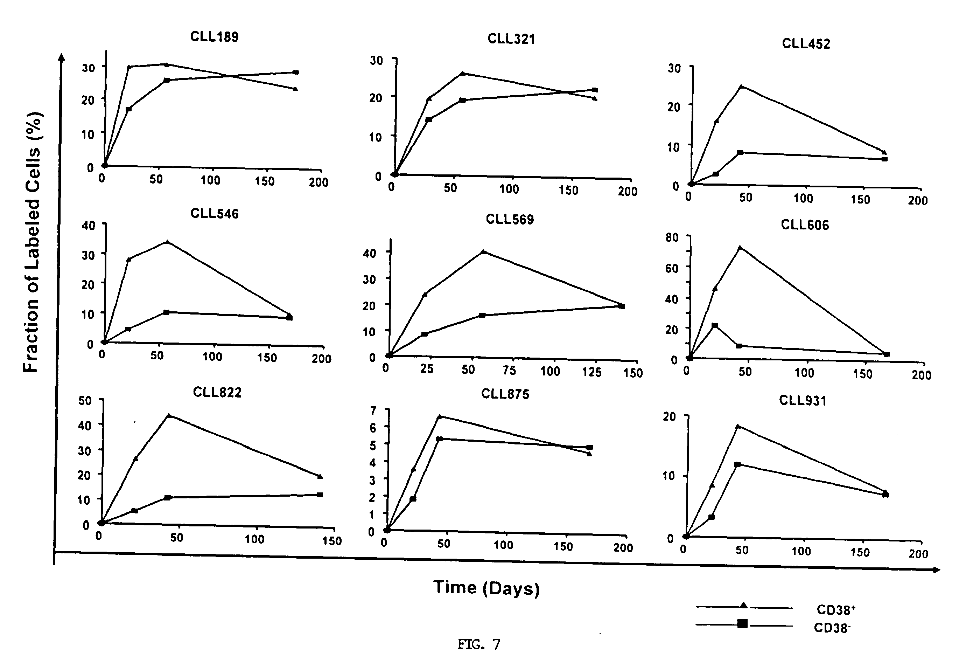

Figure 7. Kinetics of CD38⁺ and CD38⁻ CLL cells in Group A patients. Curves represent the fraction (*ƒ*) of labeled cells at the 3 time points studied. Note that different levels of 2H enrichment were observed among patients, and the graphs have different scales. Vertical dotted lines indicate the end of ²H₂O assumption. Based on the slopes of the CD38+ and CD38⁻ kinetic curves, two groups of patients were defined. Shown in this Figure, CD38⁺ cells of Group A patients rapidly achieve maximal ²H enrichment levels at the end of the labeling period; 2H incorporation in these cells subsequently falls during washout.

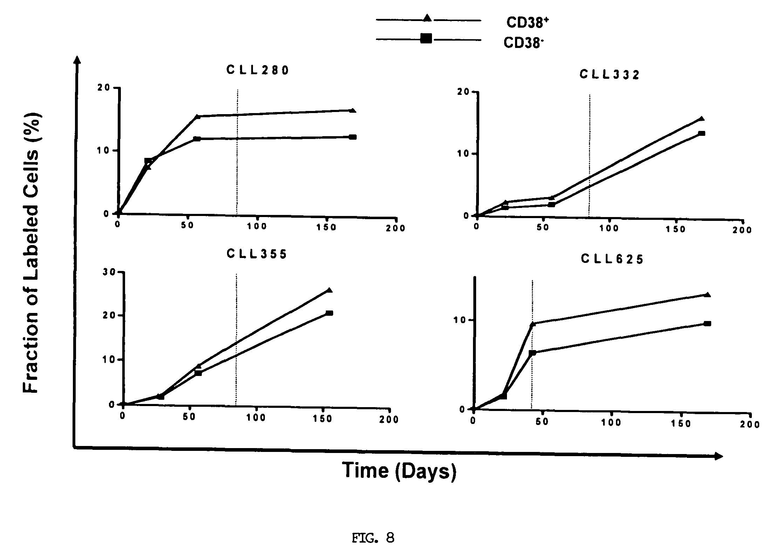

Figure 8. Kinetics of CD38⁺ and CD38⁻ CLL cells in Group B patients. Curves represent the fraction (*ƒ*) of labeled cells at the 3 time points studied. Note that different levels of ²H enrichment were observed among patients, and the graphs have different scales. Vertical dotted lines indicate the end of ²H₂O assumption. Based on the slopes of the CD38+ and CD38⁻ kinetic curves, two groups of patients were defined. Shown in this Figure, CD38⁺ cells of patients in Group B are characterized by similar kinetic of both CD38⁺ and CD38⁻ fractions with slow appearance and delayed achievement of maximal levels of labeled cells.

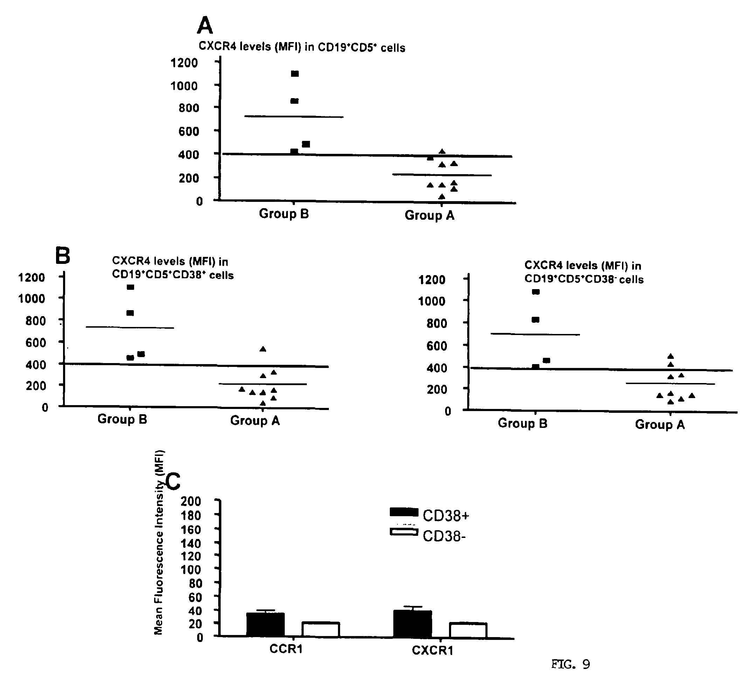

Figure 9A-9C. Chemokine receptor levels on CLL cells from the patients involved in the study. Surface membrane expression of a panel of chemokine receptors was evaluated in the 13 patients in the study. All the samples were analyzed in the same experiment. A. Group B of patients (n = 4) had significantly higher CXCR4 levels in terms of Mean Fluorescence Intensity (MFI) compared to Group A (n = 9). Comparisons of the other chemokine receptors revealed no significant differences between the two groups (not shown). B. Both CD38⁺ (left) and CD38⁻ cells (right) fractions from Group B patients showed significantly higher CXCR4 levels compared to the same two fractions from Group A. C. Of the chemokine receptor levels studied (see text for the list), only two were expressed at significantly higher densities (MFI) in CD38⁺ compared to CD38⁻ cells (CCR1, CXCR1; P < 0.05).

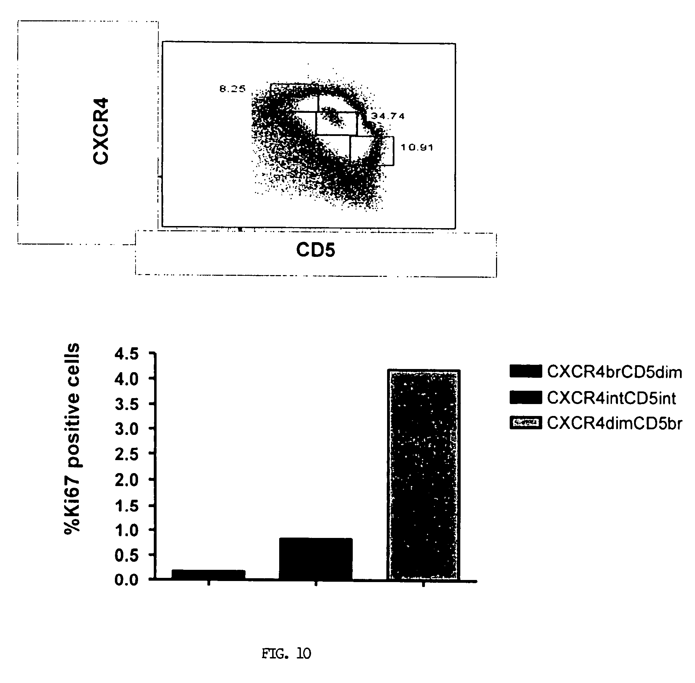

Figure 10. The CXCR4^{dim}CD5^{bright} fraction contains the majority of Ki67⁺ CLL cells. CLL clones were sorted into three fractions based on density of expression of CXCR4 and CD5 (CXCR4^{dim}CD5^{bright}, CXCR4^{int}CD5^{int}, CXCR4^{bright}CD5^{dim}). Note that there is a -20-fold increase in Ki67-expressing cells within the CXCR4^{dim}CD5^{bright} fraction of CLL cells.

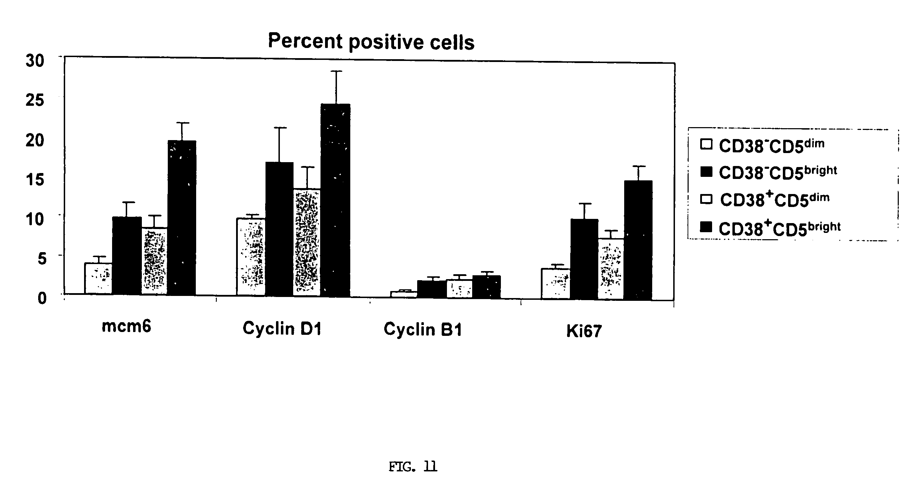

Figure 11. The CD38⁺CD5^{bright} fraction is enriched for CLL cells that have entered the cells that have entered the cell cycle. CLL clones were segregated into four fractions based on expression of CD38 and density of expression of CD5 (CD38⁻CD5^{dim}, CD38⁻CD5^{bright}, CD38⁺CD5^{dim}, CD38⁺CD5^{bright}) and then analyzed for the presence of the cell cycle-related molecules, mcm6, cyclinD1 cyclinB1 and Ki67. Note that there are more cycling CLL cells in the CD38⁺CD5^{bright} fraction.

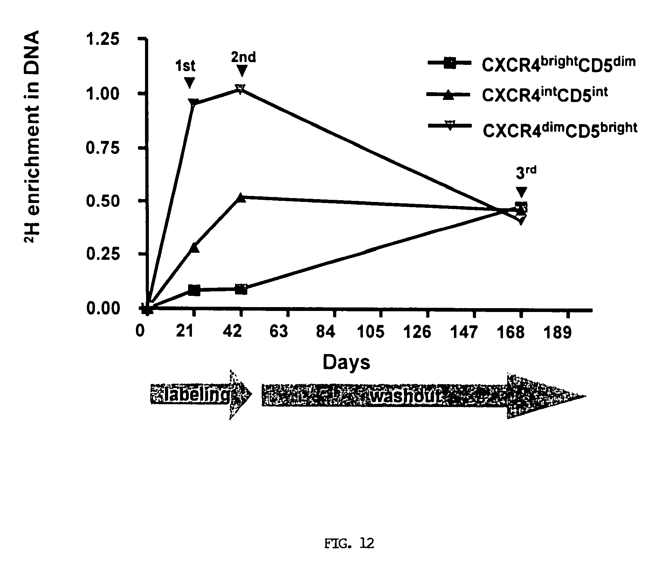

Figure 12. The CXCR4^{dim}CD5^{bright} fraction contains the majority of newly born CLL cells. CLL clones were sorted into three fractions based on density of expression of CXCR4 and CD5 (CXCR4^{dim}CD5^{bright}, CXCR4^{int}CD5^{int}, CXCR4^{bright}CD5^{dim}) and analyzed for ²H-labeled DNA as an indicator of cell proliferation. Note that there is a marked increase in ²H-labeled DNA within the CXCR4^{dim}CD5^{bright} fraction of CLL cells.

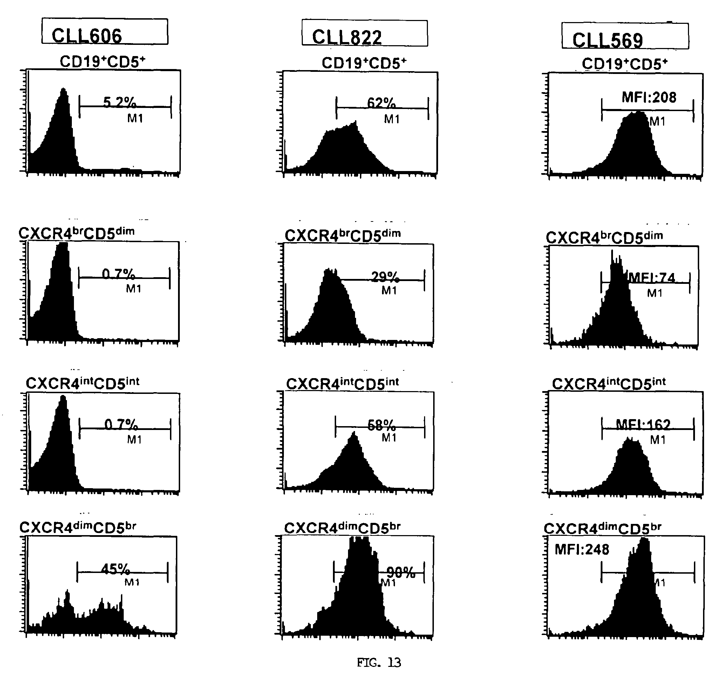

Figure 13. The CXCR4^{dim}CD5^{bright} fraction contains more CD38⁺ cells regardless of the number of CD38⁺ cells in a CLL clone. CLL clones were segregated into three fractions based on density of expression of CXCR4 and CD5 (CXCR4^{dim}CD5^{bright}, CXCR4^{int}CD5^{int}, CXCR4^{bright}CD5^{dim}) and analyzed for the presence of CD38+ cells. This was done for three CLL clones that differed in the percentage of CD38-expressing cells (CLL606 - 5.2%, CLL822 - 62%, and CLL 569 - 98%). Note that in each case, CD38-expression is increased in the CXCR4^{dim}CD5^{bright} fraction of CLL cells.

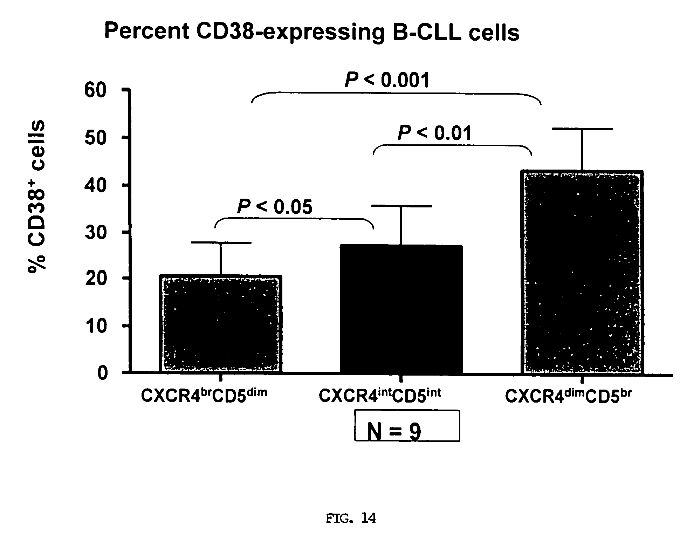

Figure 14. The CXCR4^{dim}CD5^{bright} fraction contains more CD38⁺ cells. Data represent a composite from nine CLL clones that were segregated into three fractions based on density of expression of CXCR4 and CD5 (CXCR4^{dim}CD5^{bright}, CXCR4^{int}CD5^{int}, CXCR4^{bright}CD5^{dim}) and analyzed for the presence of CD38+ cells. CD38-expression is increased in the CXCR4^{dim}CD5^{bright} fraction of CLL cells.

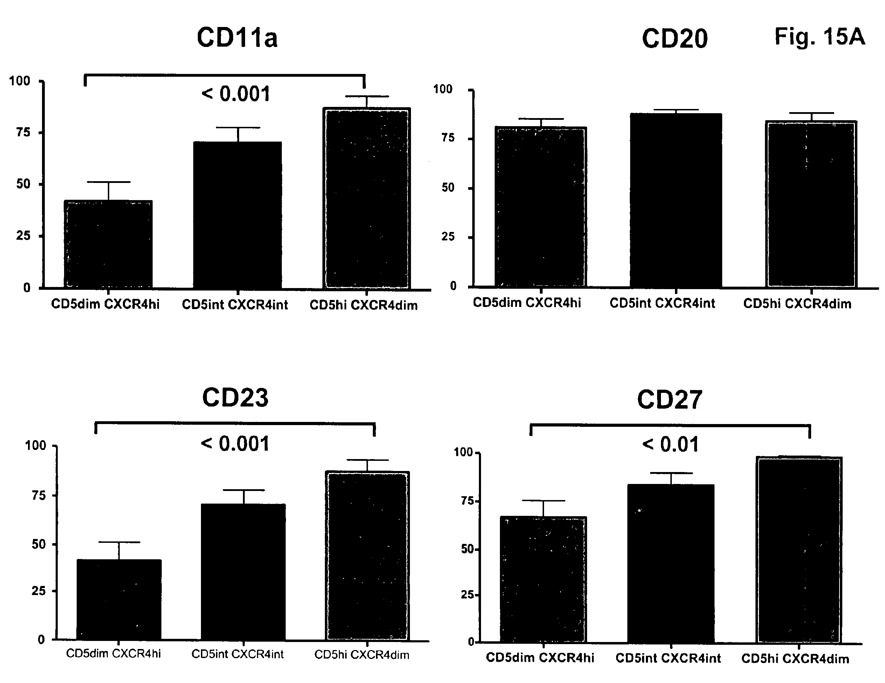

Figure 15A. The CXCR4^{dim}CD5^{bright} fraction contains more CD11a⁺ cells, more CD23⁺ cells, and more CD27⁺ cells. Data represent a composite of CLL clones that were analyzed separately for CD11a expression, CD20 expression, CD23 expression, and CD27 expression. In each instance, the CXCR4^{dim}CD5^{bright} fraction contained more CD11a⁺ cells, more CD23⁺ cells, and more CD27⁺ cells.

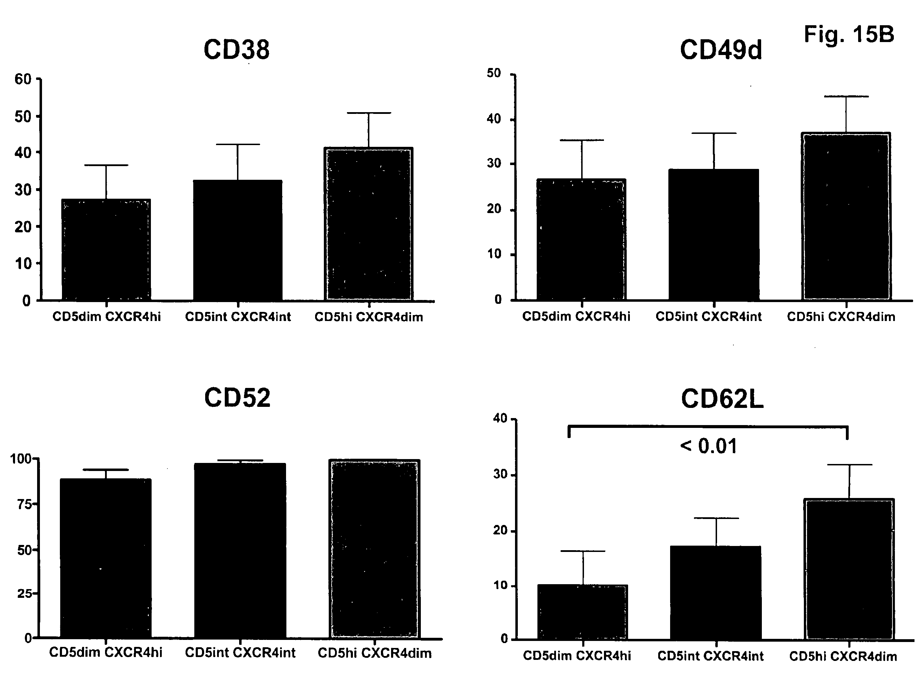

Figure 15B. The CXCR4^{dim}CD5^{bright} fraction contains more CD38⁺ cells, more CD49d⁺ cells, and more CD62L⁺ cells. Data represent a composite of CLL clones that were analyzed separately for CD38 expression, CD49d expression, and CD62L expression. In each instance, the CXCR4^{dim}CD5^{bright} fraction contained more CD38⁺ cells, more CD49d⁺ cells, and more CD62L⁺.cells.

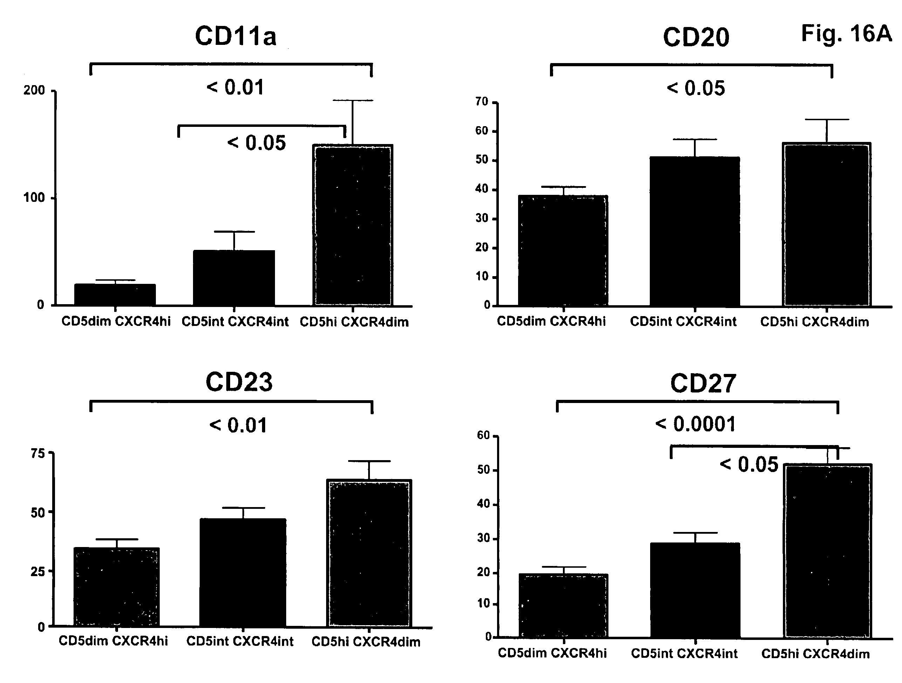

Figure 16A. The CXCR4^{dim}CD5^{bright} fraction contains more CLL cells with higher surface membrane density of, independently, CD11a, CD20, CD23, and CD27. In each instance, the CXCR4^{dim}CD5^{bright} fraction contained cells expressing, independently, CD11a, CD20, CD23, and CD27 at a higher density.

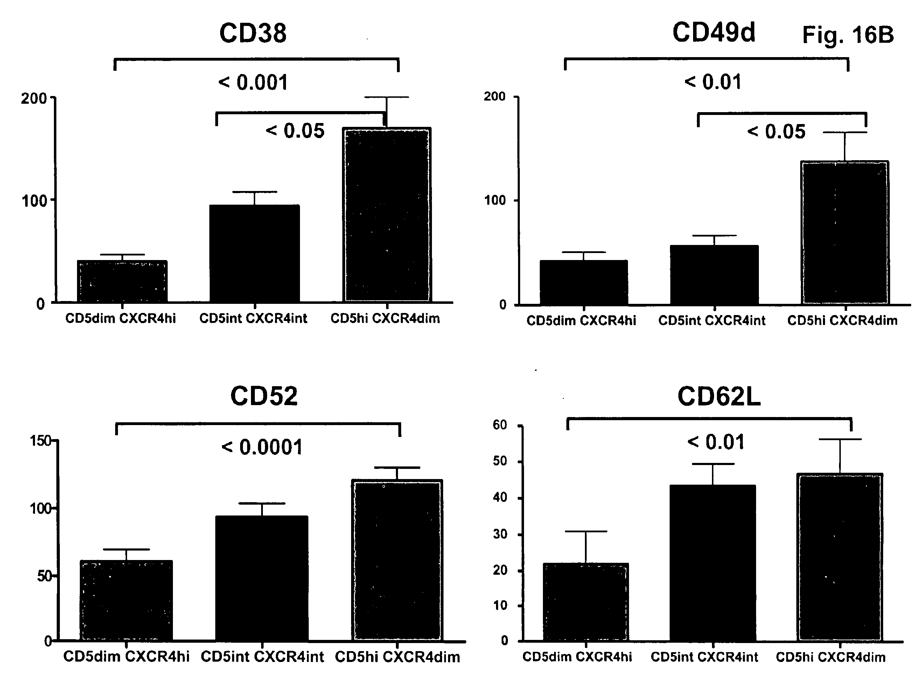

Figure 16B. The CXCR4^{dim}CD5^{bright} fraction contains more CLL cells with higher surface membrane density of, independently, CD38, CD49d, CD52, and CD62L. In each instance, the CXCR4^{dim}CD5^{bright} fraction contained cells expressing, independently, CD38, CD49d, CD52, and CD62L at a higher density.

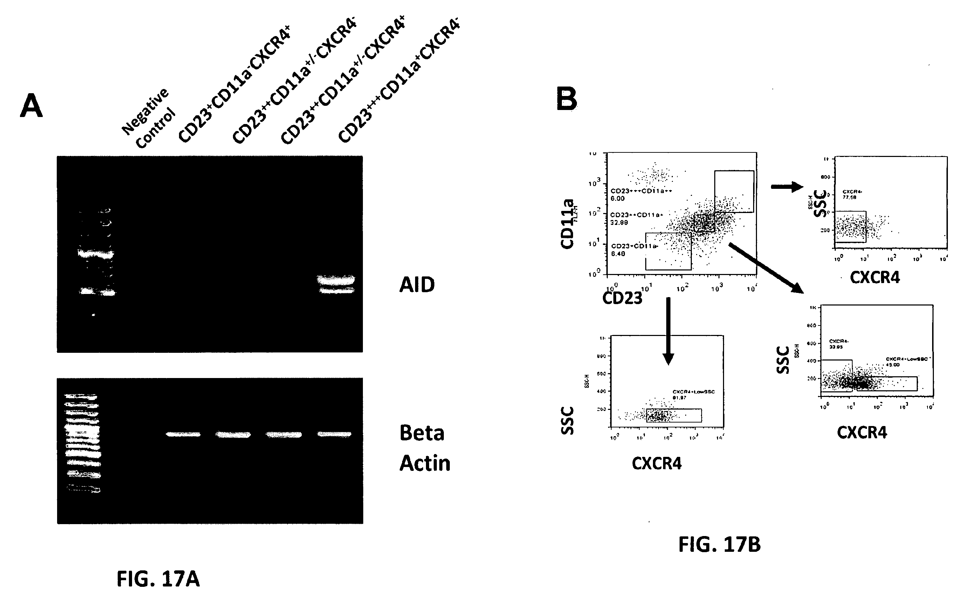

Figures 17A and 17B. This figure demonstrate that in a representative patient sample, mRNA is limited to the proliferative compartment (CD23⁺⁺⁺CD11a⁺CXCR⁻; Figure 17B).

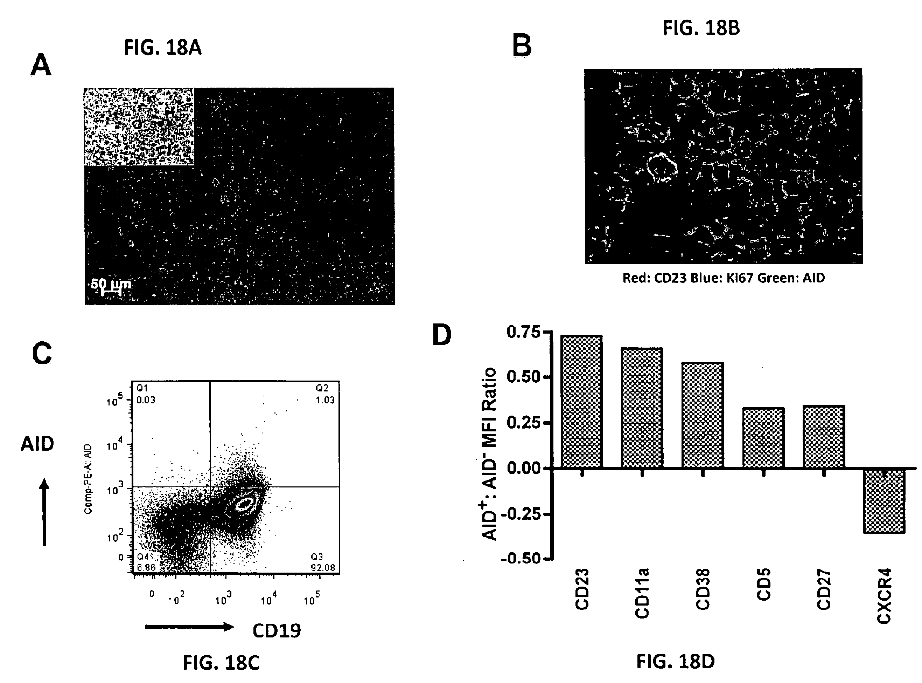

Figures 18A, 18B, 18C and 18D. AID⁺ cells, based on AID protein detected by monoclonal antibodies, are present in 50% of lymph nodes (LN) infiltrated with CLL (Figure 18A); these cells are large (Figure 18A inset), express a CLL phenotype (e.g., CD23⁺) and are predominantly in the cell cycle (as indicated by expression of Ki67; Figure 17B). Flow cytometric analysis of dispersed LN cells further confirms the presence of AID⁺ cells (Figure 18C); such cells have the phenotype of the recently divided cells found within peripheral blood (Figure 18D).

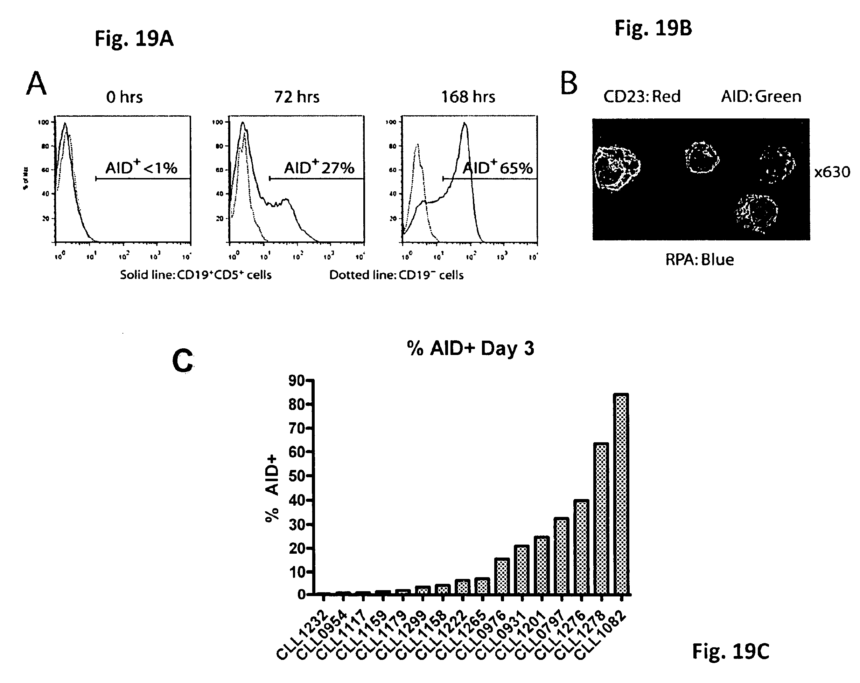

Figures 19A, 19B and 19C. It is known that culture of CLL cells activated by anti-CD40 in the presence of with irradiated CD32-transfected fibroblasts and IL-4 upregulates AID mRNA; Figure 19A (FACS analysis) and 19B (immunofluorescent microscopy) demonstrate a parallel increase in AID protein. A series of 16 patients shows that the extent of such upregulation is highly variable (Figure 19C).

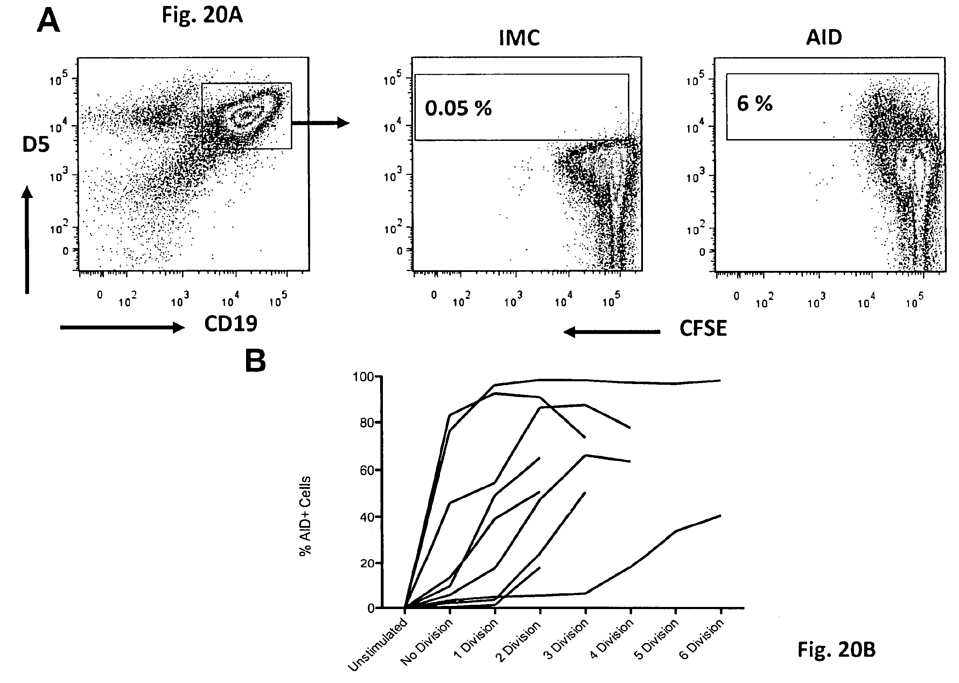

Figure 20A and 20B. Using the dye CFSE to track CDS⁺CD19⁺ cell division in this same culture system, AID protein expression is increasingly upregulated on the most divided cells (Figure 20A); this finding is consistent in all samples where dividing cells are found although the rate and extent of AID upregulation varies (Figure 20B). Thus, the proliferative compartment is indeed the major source of AID+ cells.

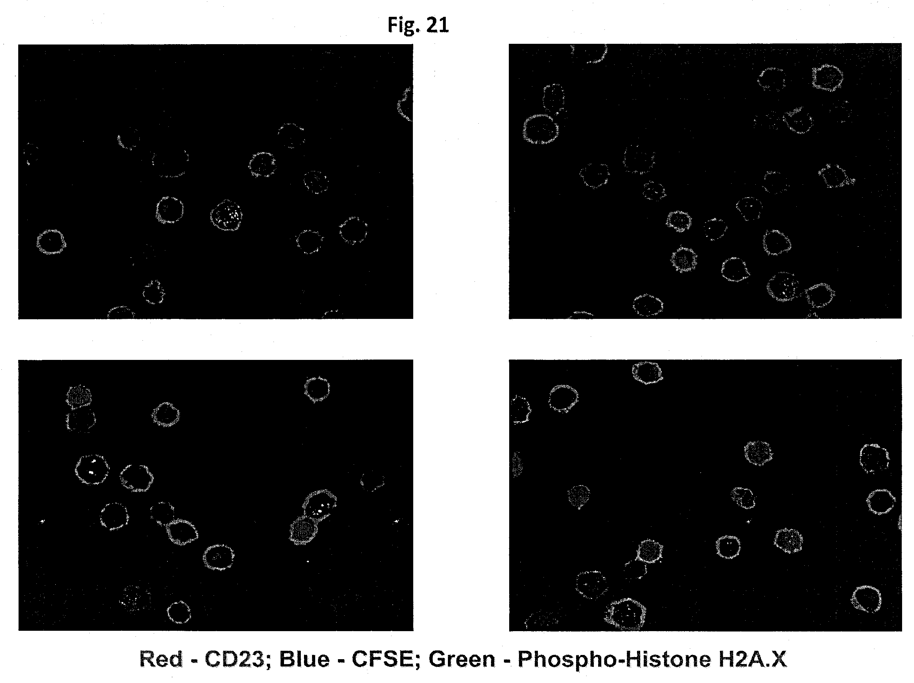

Figure 21. AID activity is known to cause double strand breaks (DSBs) within DNA, for example in immunoglobulin switch regions during class switch recombination. DSBs (visualized using an antibody to phospho-histone H2A.X) are much more marked in the most divided (CD23⁺CFSE^{dim}) cells compared to no/minimally divided (CD23⁺CFSE^{bright}) cells (Figure 21).

### Detailed Description of the Invention

To the best of our knowledge, all therapies for CLL are based on the premise that every cell of the clone is equally dangerous to the patient and therefore effective therapies must eliminate every cell to affect a cure. This view is supported by the facts that, despite being able to eliminate all detectable cells post certain chemo-immunotherapies (e.g., fludarabine + cyclophosphamide + Rituxan), all patients eventually relapse. Furthermore, the only therapy that does result in apparent cures is allogeneic bone marrow transplantation that appears to require a certain level of ongoing graft vs. host and graft vs. tumor activity to be effective. Both the ineffectiveness of combined therapy and the need for an ongoing immune reaction post allo-transplantation suggest that there are residual cells that remain after induction therapy and that can on occasion be continuously eliminated by an ongoing allo-immune reaction. These might be leukemic stem cells that give rise to new clonal members that can evolve more dangerous somatic mutations over time, i.e. clonal evolution. However the existence of leukemic stem cells in CLL is not a pre-requisite for our therapy to be effective.

That clonal evolution occurs and is responsible for clinical progression is supported by findings that certain defined chromosomal abnormalities that exist in CLL are not seen in all members of the clone and often are not detected until the patient has had the disease for many years. Furthermore, these abnormalities occur more frequently as the absolute lymphocyte count increase and as clinical decompensation occur.

Activation-indcued cytidine deaminase (AID) is an enzyme that is necessary and sufficient to initiate IGV gene mutations and isotype class switching in normal B lymphocytes. The action of AID is usually restricted to the Ig loci and a few others. However in certain disease, AID appears to act outside of these restricted areas and thereby induces genomic abnormalities that can cause or exacerbate cancer. A relationship between AID and CLL evolution and progression could exist.

The novel form of therapy which we propose is based on the hypotheses that: [1] those cells that proliferate (proliferative compartment), which might (but need not) be progeny of leukemic stem cells, are the most dangerous members of the leukemic clone because these are the only cells that can develop new permanent DNA abnormalities that could lead to more aggressive clonal variants, [2] these proliferating cells are present at low numbers throughout the disease but may be increased with disease worsening and/or after inductive therapy, especially at the early stages of disease relapse, [3] elimination of these cells, either during inductive (initial or later) or consolidation therapy, will protect the patient from the emergence of more deleterious subclones, [4] this approach may convert CLL into an even more chronic disease and/or lead to an eventual cure, and finally [5] this therapeutic approach will be much less toxic than current immune- and chemo- therapies that do not target CLL-cell specific and human B-cell specific targets. This approach might result in only a minor fall in the absolute number of leukemic cells (at least initially) and therefore will either permit existing, less dangerous clones to persist or permit such clones to eventually die spontaneously or after other forms of therapy.

We propose that by targeting a series of surface membrane antigens expressed preferentially on cells of the proliferative compartment with reagents (Abs, peptides, aptamers, T or natural killer cells with chimeric bispecific antibody receptors, or other yet to be defined specific molecules) reactive with these antigens will lead to the death of newly generated cells that could lead to clonal evolution and disease worsening. Such therapy may need to be delivered chronically.

By analyzing co-expression of a series of surface membrane molecules, e.g., CD5, CXCR4, CCR7, CD11a, CD19, CD20, CD23, CD27, CD38, CD48, CD49d, CD52, CD62L, and CD100, one can identify those cells in a CLL leukemic clone that divide the most vigorously ("proliferative compartment"). See Figure 1 for graphic representation of this and other fractions. We have identified cells in the proliferative compartment based on subsettting the leukemic clone using some of the above markers and then quantifying enrichment of ²H into newly synthesized DNA in specific fractions of leukemic cells from patients that have consumed ²H₂O (a direct in vivo measure of cell proliferation). Furthermore, we have used enrichment of leukemic cells expressing the intracellular marker Ki-67 as another indicator of cells belonging to the proliferative compartment. By using a combination of markers expressed by cells enriched in 2H-labeled DNA and/or Ki-67-expression, we have identified an extended surface membrane phenotype that can be used to identify and quantify, in a diagnostic manner, the proliferative compartment. In addition certain combinations of these markers can be used as unique targets of a novel form of therapy designed to eliminate, preferentially, the proliferative compartment.

By targeting the proliferative compartment with reagents that bind a combination of the above markers, one will be able to preferentially and effectively eliminate these cells, some of which are accumulating new genomic mutations that could lead to disease progression and escalation. Any individual or combination of markers could be used for this purpose. Based on our current data, the individual targets that would encompass the largest fraction of the proliferating compartment are CD20, CD11a, CD23, CD27, CD38, CD48, CD49d, CD52, CD62L, and CD100. Any combination of targets would enhance targeting of the proliferative compartment; at this juncture, the combination that would encompass the largest fraction of the proliferative compartment are (i) CD 19 and CD11a, (ii) CD 19 and CD23, (iii) CD19 and CD27, (iv) CD19 and CD38, (v) CD19 and CD48, (vi) CD19 and CD49d, (vii) CD 19 and CD52, (viii) CD 19 and CD62L, and (ix) CD 19 and CD 100; (x) CD20 and CD11a, (xi) CD20 and CD23, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD23 and CD52, (xxv) CD23 and CD62L, and (xxvi) CD23 and CD100.

Furthermore, by choosing at least one marker that is expressed solely or primarily on human B lymphocytes (the precursor lineage of leukemic CLL cells), specific targeting for CLL cells will be assured. Preferential targeting of the proliferative compartment of CLL cells and B cells could be achieved in several ways.

For instance, a bi-specific antibody with reactivity with two B-cell specific or biased molecules expressed at higher density on cells of the proliferative compartment or in higher numbers in the proliferative compartment (such as but not limited to CD20 and CD23) would bind preferentially to that fraction of leukemic and normal cells that are actively dividing or have just divided. A similar approach could incorporate one B-cell specific molecule and another molecule that is not B-cell specific but that is expressed on more proliferating CLL cells and/or in higher density, thereby again imparting preferential targeting and elimination of these cells. Non-exclusive examples would include reagents reactive with CD20 + CD52, CD23 + CD52, and CD23 + CD11a.

An alternative approach would be to administer simultaneously, or in tandem, two distinct standard bivalent, mono-specific mAbs, each with specificity for two of the B-cell specific molecules listed above.

The mAb reagents used could be either native molecules of the types mentioned above or these antibodies conjugated with any one of a series of toxins or radioligands. The former reagents would potentially induce apoptosis or permit antibody-dependent cytotoxicity or complement-mediated cytotoxicity; the latter reagents would serve as delivery agents to preferentially target the proliferative compartment with a toxic moiety of the types mentioned or others that might be developed in the future.

Targeting of the proliferative compartment could be done at several points in disease evolution. In one embodiment described herein, the therapy can be initiated at the time of diagnosis or early in the course of disease. In this instance, targeting the proliferative compartment would likely prevent or at least delay significantly the emergence of more dangerous clonal variants by diminishing or eliminating the fraction of the leukemic clone that can develop permanent DNA abnormalities during the replication of DNA; the development of new DNA lesions is an essential step in disease progression. Although possibly not curative, this approach could lengthen the time to treatment interval substantially, converting the disease into an even more chronic condition.

In another embodiment described herein, the therapy can be initiated after initial therapy with other therapies. Although treatment regimens are emerging that can be effective in eliminating the numbers of leukemic cells in the blood and solid tissues, invariably patients relapse. Furthermore, there is no direct evidence at this point that any of these regimens extends the life span of the patient. Therefore, additional treatments are given to patients to "consolidate" remissions and lengthen the time to relapse. Unfortunately, many/most of these consolidation therapies are not CLL-specific and therefore often worsen the hematopoietic and immune function of patients, making them much more susceptible to infection and diminishing their overall quality of life because of persistent anemia or low clotting elements (thrombocytopenia, etc).

Because the proliferative compartment, at the time of numerical clonal depression after therapy as well as at the time of relapse, can be usually larger than prior to therapy, the effectiveness of targeting this compartment at these junctures would be enhanced. Furthermore, since targeting, using this approach, can be made completely B-cell specific (e.g., using a bispecific mAb reactive with both a B-cell specific surface membrane molecule and another molecule over-expressed on dividing/divided leukemic cells), this therapeutic approach would minimize the off-target side effects seen with many other consolidation therapies.

The agents described in the present specification can also be used as a remission-inducing therapy. Although this therapeutic approach is designed to preferentially eliminate the proliferative fraction of a leukemic CLL clone, it could be effective at inducing therapeutic remissions. These remissions however would take longer to manifest in the periphery (as measured by CLL cell counts in the blood) because the proliferative compartment in most CLL clones is small (usually -0.1 - <2.0% cells dividing per day). Nevertheless, this appraoch would eliminate the cells that are precursors of the majority of the clone detected by routine measures (i.e., not considering the proliferative or resting status of leukemic cells).

With respect to diagnostic possibilities, by using a series of monoclonal antibodies (mAbs) or other reagents specific for the CD antigens identified in multi-color flow cytometric analyses, one can identify and quantify the numbers of cells in the proliferative compartment. In addition, by analyzing the sizes of these compartments among a cohort of CLL patients, one can define a typical numerical and proportional range for each compartment. This information would allow a comparison of the sizes of these compartments in an individual patient with a standard range among a large cohort of patients.

Values exceeding these ranges on a single determination within an individual patient or changes in values in the same patient over time could be used as indicators of disease aggressiveness and progression. For example, an increase in the size of the proliferative compartment could signal an increase in the proliferative rate of a leukemic clone. Such a clone would be more likely to have individual leukemic cells that develop new DNA abnormalities, indicating that clonal evolution is a more likely possibility and therefore a change in the aggressiveness of the disease is imminent or has occurred. Such a change could be used as an indicator that therapy is now warranted or at least should be considered.

The same approach can be used after therapy as a monitor of the re-emergence of the proliferative fraction of the leukemic clone and at various time points after relapse. Again, numbers of cells exceeding a typical range for a cohort of recovering or relapsing patients or a value in the same patient that increases over time would be indicators of disease progression and increased aggressiveness. This increase also could be used as an indicator that therapy is now warranted or at least should be considered.

This invention will be better understood from the Experimental Details which follow. However, one skilled in the art will readily appreciate that the specific methods and results discussed are merely illustrative of the invention as described more fully in the claims which follow thereafter.

### Experimental Details

### Example 1

B-cell chronic lymphocytic leukemia (CLL) is a relatively common and incurable adult disease of unknown etiology. The tumor mass is an expansion of a CD5⁺ B lymphocyte clone whose members share the same B-cell receptor (BCR) antigen-binding domains. The vast majority of circulating CLL cells is non-proliferating lymphocytes (1, 23, 23).

Nevertheless, a small proliferative compartment does exist (12, 41) and lymph nodes and bone marrow contain aggregates of activated, dividing cells (called "proliferation centers" or "pseudofollicles") that apparently represent the preferred site of relapse (24). In addition, accessory signals delivered by bystander cells in the microenvironment of solid tissues are essential for neoplastic cell survival and expansion (25), suggesting that clonal accumulation requires survival signals that rescue leukemic cells from death. Therefore, CLL, like other leukemias and lymphomas, exhibits a dynamic interaction between birth and death of cells (12).

CD38 is a surface membrane bound ectoenzyme that functions as a receptor (26, 27). Expression of CD38 on normal B cells depends on the stage of cell maturation and can be induced upon stimulation (8). The percentage of clonal members expressing surface membrane CD38 has prognostic significance in CLL (7, 28), and this prognostic value appears linked to its capacity to promote leukemic cell survival (9, 29). Moreover, within each CLL clone, cells expressing CD38 are enriched in expression of Ki-67, a cell cycle-restricted marker, suggesting that CD38+ cells represent a cycling subset (10).

Deuterium (2H) incorporation into newly synthesized DNA is an established measure of DNA replication (30, 31). We used a non-radioactive, stable isotopic labeling method to measure CLL cell kinetics *in vivo.* Patients drank an aliquot of deuterated water (²H₂O) daily for 42-84 days (Figure 2), and ²H incorporation into the deoxyribose moiety of DNA of newly divided CLL cells was measured by gas chromatography/mass spectrometry, during and following the labeling period. Birth rates were calculated from the kinetic profiles. Using this approach, CLL cells were found to proliferate *in vivo* at rates ranging from 0.1% - 1.7% of the clone per day, and patients with higher birth rates appeared at risk of more active disease (12).

We have used *in vivo* ²H labeling to better understand the phenotype of the most actively proliferating cells within each CLL clone (see model in Figure 1). In 13 patients (Table 1), ²H enrichment in genomic DNA was compared between CD38+ and CD38- cells of 13 patients and indicated intra-clonal heterogeneity in cellular proliferation, with CD38+ cells proliferating more rapidly. As illustrated in Table 2, a two-fold higher percentage of newly produced cells was found in the CD38+ compared to the CD38- compartment at both time points during labeling period (16.5% ± 3.9 vs. 7.3% ± 1.8 at 1st time point, P <0.01; 25.9% ± 5.4 vs. 11.2% ± 1.7 at 2nd time point, P = 0.013). Moreover, when data obtained during the labeling period were analyzed on an individual patient basis, a larger fraction, *ƒ* was found in the CD38+ population of all but one patient (CLL280) at one (the 1st) time point, with the maximum numbers ranging from 6.6% to 73% (CLL 875 and 606, respectively).

Ratios of percent newly labeled cells (*ƒ*) in CD38+ to CD38- fractions are presented in Figure 3. There is approximately a 2.5 times greater percentage of newly synthesized cells in the CD38+ fraction; this is achieved by time point 1 and maintained until the end of ²H₂O administration (time point 2). Of note, observed differences did not correlate with the number of CD38+ cells in individual CLL clones prior to fractionation (range 1% - 98%; Table 1). For example, when analyzing data from cells taken at the 1st time point, CD38+ to CD38- labeled fraction ratios were >2 in 6 patients (CLL 452, 546, 569, 606, 822, 931; Table 2) and the percent of CD38+ cells in these clones ranged from 7% to 98% (Table 1).

Ki-67 is a cell cycle related molecule (32, 33), and Ki-67-expressing cells are enriched in CD38+ fractions of individual CLL clones (10). As illustrated by CLL822, the Ki-67+ fraction of this clone (5.5%; upper right panel) is markedly enriched in CD38+ cells (83%; Figure 4, lower right panel). Similar enrichments of CD38+ cells in the Ki-67+ fractions were found in all patients tested (Figure 5).

To directly prove that Ki-67 expression represents proliferating cells in CLL, CD19+CD5+ cells from CLL452 were sorted into four fractions based on expression of Ki-67 and CD38 (Figure 6A). Consistent with phenotypic data, *ƒ* was higher among cells expressing Ki-67 at both time points during the labeling period, with a hierarchy of incorporation being CD38+Ki-67+ > CD38-Ki-67+ > CD38+Ki-67- > CD38-Ki-67-. Of note, the CD38+Ki-67+ fraction contained ~50% newly divided cells and the Ki-67+ cells represented a highly proliferating subset, defined by ²H-incorporation in DNA. Similar findings were obtained from CLL625 (Figure 6b). In this case, 70% of cells in the CD38+Ki-67+ fraction were new and already at plateau by 21 days of labeling, suggesting a minimum estimated cellular turnover rate of 3.3% per day (70%/21 days), ~2-30 fold higher than the rates previously observed for whole CLL clones (12). The plateau observed at ~70% suggests 30% of the Ki-67+ cells either are not dividing or are dividing at an extremely slow rate.

There were also clear differences in CD38+ and CD38- kinetics among patients (Figure 8). For most cases (Group A, Figure 7), CD38+ cells rapidly achieved a maximal fraction of ²H-containing cells at the end of the labeling period. These fractions exceeded, in some instances dramatically (e.g., CLL 452, 546, and 822), those reached by CD38- cells. ²H incorporation in CD38+ cells then fell during washout. (Patient CLL606 was peculiar because of the loss of 2H-labeled CD38- cells before the end of the labeling period; the kinetics for CD38+ cells, however, resembles those observed in the other members of Group A.)

However, the kinetics of patients 280, 332, 355 and 625 (Figure 8, Group B) differed significantly from the 9 patients mentioned above. The fraction of ²H-labelled CD38+ cells of these patients reached maximum levels only at the end of the washout period. Moreover, CD38+ and CD38- fractions in these patients had very similar kinetics, compared with the more heterogeneous and usually clearly divergent curves of the other patients. These observed differences were quantified. The slopes recorded between time point 2 and 3 were concordant positive in Group B and non-concordant positive in Group A.

Differences in ²H labeling of CD38+ and CD38- cells could relate to the time cells take to exit solid tissues where division and 2H incorporation into DNA most likely occurred. Because chemokines and their receptors are involved in cell retention, migration, and homing (34-38), we analyzed several chemokine receptors in all 13 patients (Figure 9). CLL cells were evaluated for CCR1, CCR2, CCR4, CCR5, CCR7, CXCR1, CXCR3, CXCR4, and CXCR5, both as percent positive cells as well as mean fluorescent intensities (MFIs). The latter was necessary because chemokine receptors such as CXCR4, CXCR5 and CCR7 are expressed on virtually every cell in a CLL clone (34), albeit over a range of densities. Only CCR1 and CXCR1 were expressed at greater densities in the CD38+ fractions in all 13 CLL clones studied (P <0.05; Figure 9C).

We then compared data for patients in Group A with those in Group B, since the latter had a slower appearance of ²H-marked cells in the blood, suggesting longer retention in solid tissues. No differences were found in the percentage of chemokine receptor-expressing cells in Group A vs. Group B (not shown).

However, when analyzed for cell surface density (MFI), a significant difference in CXCR4 expression was seen between the two groups (MFI Group B: 720 ± 160 vs. Group A: 238 ± 47; P = 0.01; Figure 9A). In addition, all 4 patients in Group B had a CXCR4 MFI higher than 400, compared to only 1/9 in Group A. Furthermore, consistent with the observation that in Group B CD38+ and CD38- fractions had similar kinetics (Figure 8), both CD38+ and CD38- subsets exhibited higher CXCR4 densities compared with the same fractions in Group A (Figure 9B). Thus higher CXCR4 densities were a property of CLL clones exhibiting delayed appearance in the periphery.

Clinical data for the patients in this report are provided in Table 1. Of the 13 patients studied, 8 experienced progression in Rai stage, required treatment, or had a fatal course. This was the case for all patients in Group B (CLL 280, 332, 355, 625) but only for 4/9 (44.4%) of those in Group A. Group B patients had a shorter, albeit not statistically significant, median time to treatment compared to Group A (57.6 vs. 119.1 months; P = 0.06). Also, all Group B patients had involvement of a lymphoid organ detectable by physical examination, while this was the case again for only 4/9 patients of Group A. Mean WBC counts averaged over the course of the study for Group B patients were significantly higher (P = 0.044) than those of Group A. Lastly, only one patient (CLL280) in Group B had a leukemic clone expressing a mutated IGHV. Among the IGHV mutated cases in the entire cohort, CLL280 is the only patient who progressed in terms of a change in Rai stage, had spleen and liver involvement, and the highest WBC and ZAP-70+ CLL cell counts.

Thus, inter-clonal heterogeneity exists, with two patient groups showing distinct kinetic patterns and significantly different CXCR4 levels. The group with higher CXCR4 levels (4/13 patients) showed a delayed appearance of ²H-labelled CD38+ cells in blood and seemed at a higher risk for lymphoid organ infiltration and inferior outcome, thereby suggesting relationships between CLL kinetics, expression of a molecule involved in CLL cell retention and trafficking to solid tissues (15, 39) and prognosis (40).

In follow-up studies, we have used CXCR4 expression to further dissect the CLL proliferative compartment by analyzing those fractions expressing various levels of CXCR4 in conjunction with CD5, a known B-cell activation antigen) and with Ki-67 and MCM6, two cell cycle related markers. We observed a relationship between CXCR4 and CD5 such that CXCR4^{dim} cells were CD5^{bright} and vice-versa, with the majority of the cells falling in an intermediate category (CXCR4^{int}CD5^{int}) (Figure 10). The CXCR4^{dim}CD5^{bright} subpopulation contained significantly more Ki67+ and MCM6+ cells (Figure 11). Based on this observation, CLL cells were sorted into CXCR4^{bright}CD5^{dim}, CXCR4^{int}CD5^{int}, CXCR4^{dim}CD5^{bright} fractions at three time points, and ²H was measured in an equal amount of DNA from each fraction. A hierarchy of ²H content in the fractions was found: CXCR4^{dim}CD5^{bright} > CXCR4^{int}CD5^{int} > CXCR4^{bright}CD5^{dim}. These differences were statistically significant during the labelling period (1^{st} and 2^{nd} time point).

*In vitro* studies have suggested that CXCR4 has a crucial role in trafficking and survival of CLL cells, and it is emerging as a powerful predictor for bone marrow infiltration and clinical evolution. CXCR4 surface expression is downregulated after encountering its ligand SDF-1. Our data indicate a relationship between CXCR4 levels and CLL kinetics (Figure 12). Moreover, since CLL cell subpopulations defined by relative CXCR4 and CD5 levels differ in the number of proliferating cells, the CXCR4^{dim}CD5^{bright} markers can be used to define cells that have emigrated recently (proliferative compartment) and combined CXCR4^{bright}CD5^{dim} expression can be used to define cells that left earlier from the solid tissue in which they divided. The results strongly suggest the important role of the stroma and SDF-1-producing cells for CLL cell proliferation, *in vivo,* providing indications for disease control strategies.

Finally, we have recently analyzed the other cell surface molecules expressed by the proliferative compartment and the resting/re-entry compartment. Using the number of clonal members expressing CD38 and those members expressing different densities of CD5 and CXCR4, we have further characterized these two important CLL subfractions. Regardless of the number of CD38+ cells in a CLL clone, those cells with the CXCR4^{dim}CD5^{bright} phenotype are enriched in CD38-expressing cells (Figure 13). A composite of such data is shown in Figures 14. The CXCR4^{dim}CD5^{bright} fraction is also enriched in the number of cells expressing CD11A, CD23, CD27, CD38, CD49d, CD52 and CD62L (Figure 15A and 15B). Furthermore, this fraction also contains cells expressing higher surface membrane densities of CD49d (Figure 16) as well as cells independently expressing higher densities of CD11A, CD20, CD23, CD27, CD38, CD49d, CD52 and CD62L (Figure 16A and 16B).

**Table 1: Clinical and laboratory features of the CLL patients involved in this study**

| | % CD38* | % ZAP-70* | Cytogenetic abnormalities (% cells) | | | | *IGHV mutation* status^{#} | Lymph node/Spleen/ Liver enlargement ^ | Rai stage at diagnosis and when ²H₂O study began | Status | Time to first treatment (months)* | Mean WBC (beginning-end of study)^{$} |
|---|---|---|---|---|---|---|---|---|---|---|---|---|
| | | | Δ11q22 | Tri12 | Δ13q14 | Δ17p23 | | | | | | |
| CLL189 | 2 | 7 | <5 | <5 | 70 | <5 | M/UM | + / - / - | 0-IV | Expired | 53 | 107 (76-175) |
| CLL280 | 1 | 29 | <5 | <5 | 99 | <5 | M | - / + / + | 0-II | Alive | 108 | 58 (61-75) |
| CLL321 | 10 | 95 | <5 | 59 | <5 | <5 | UM | - / - / - | 0-0 | Alive | None | 73 (102-90) |
| CLL332 | 66 | 88 | na | <5 | 93.5 | <5 | UM | + / - / - | I-I | Alive | 28 | 74 (na-87) |
| CLL355 | 25 | 18 | 68 | <5 | 25 | <5 | UM | + / - / - | I-I | Expired | 88 | 228 (71-310) |
| CLL452 | 36 | 48 | na | na | na | na | M/UM | + / - / - | 0-I | Alive | 36 | 79 (63-105) |
| CLL546 | 7 | 6 | 0 | 0 | 78 | 0 | M | - / - / - | 0-0 | Alive | None | 32 (27-35) |
| CLL569 | 98 | 40 | 0 | 0 | 0 | 0 | UM | - / + / - | II-II | Alive | None | 73 (52-95) |
| CLL606 | 8 | 5 | 0 | 0 | 68 | 0 | M | - / - / - | 0-0 | Alive | None | 29 (30-26) |
| CLL625 | 54 | 57 | 80 | 0 | 0 | 0 | UM | ++ / - / - | II-II | Alive | 17 | 297 (245-261) |
| CLL822 | 22 | 56 | 0 | 0 | na | 0 | UM | - / + / - | 0-III | Alive | 108 | 43 (30-75) |
| CLL875 | 14 | 18 | 0 | 0 | 0 | 0 | M | - / - / - | 0-0 | Alive | None | 15 (15-15) |
| CLL931 | 63 | 49 | na | na | na | na | UM | - / - / - | 0-0 | Expired | None | 20 (17-28) |

| | | | | | | | | | | | | |
|---|---|---|---|---|---|---|---|---|---|---|---|---|
| na: Data not available #: ≤ 2% difference from the germline gene defines a patient as *IGHV unmutated* (UM); >2% difference defines a patient as *IGHV mutated* (M). ^: Lymph nodes: + 1 - 1.5 cm; ++ 1.5 - 3 cm. + for spleen and liver defines them as palpable by physical examination or enlarged on imaging (ultrasound or CT scan). Only data 6 months before or after the study are included. *: Indicates patients that received the first treatment for clinical progression of CLL in months in relation to diagnosis. $: Mean WBC count recorded during ²H₂O protocol period; in brackets are values at the beginning and end of the protocol. Note that WBC data for CLL569 were incomplete (only beginning and end values were available) and WBC count of CLL332 at the beginning of the study was not available. | | | | | | | | | | | | |

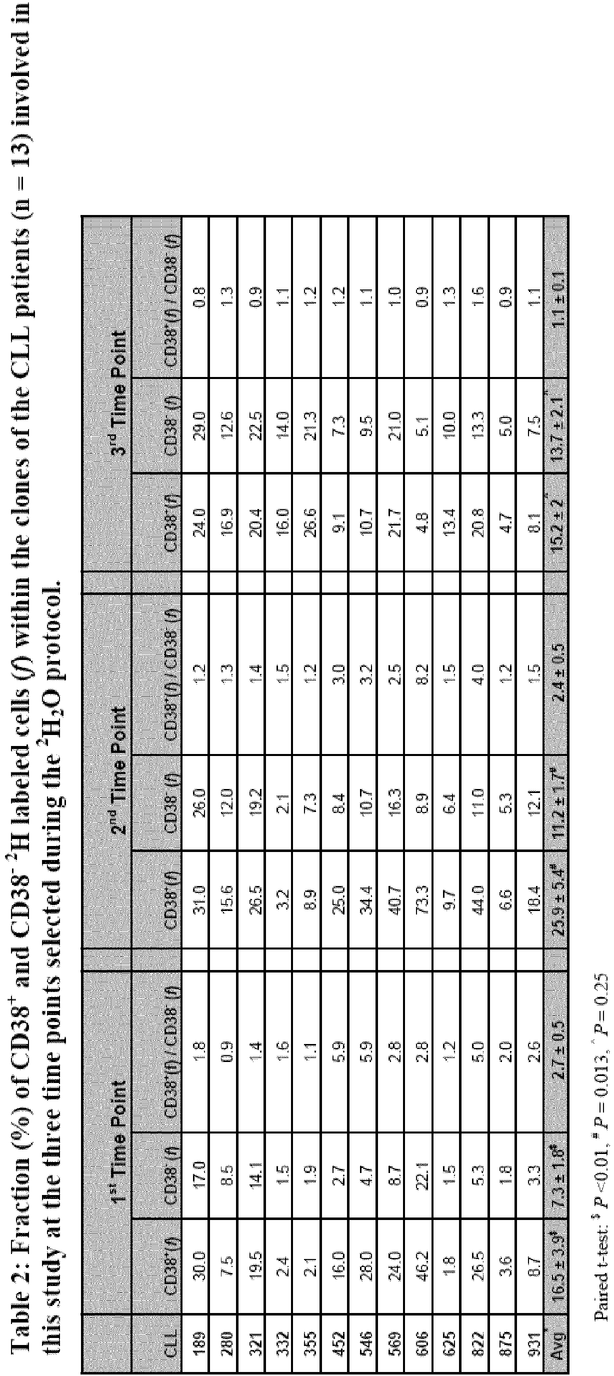

### Example 2

During the disease course of CLL, ongoing genetic changes are evident within the leukemic clone, and such changes are associated with disease progression. Activation-induced cytidine deaminase (AID), an enzyme required for immunoglobulin class switching and somatic hypermutation in B cells, is a candidate enzyme for causing such changes.

Depending upon the detection method peripheral blood CLL cells express mRNA for AID in between 40% to 100% of patients but at very low levels within a very minor population of cells (~0.01-1.0%). Because of the presence of AID in such a small number of cells, we hypothesized that those cells are contained within the recently divided subset ("proliferative compartment"). Figure 17A demonstrates, in a representative patient sample, that mRNA is limited to this subset (here defined as CD23⁺⁺⁺CD11a⁺CXCR⁻; Figure 17B).

Dividing cells in CLL are principally found within bone marrow and secondary lymphoid tissue. Based on the above data, these are candidate cells for AID protein expression. Indeed, AID⁺ cells, based on AID protein detected by monoclonal antibodies, are present in 50% of lymph nodes (LN) infiltrated with CLL (Figure 2A); these cells are large (Figure 18A inset), express a CLL phenotype (e.g., CD23⁺) and are predominantly in the cell cycle (as indicated by expression of Ki67; Figure 18B). Flow cytometric analysis of dispersed LN cells further confirms the presence of AID⁺ cells (Figure 18C); such cells have the phenotype of the recently divided cells found within peripheral blood (Figure 18D).

It is known that culture of CLL cells activated by anti-CD40 in the presence of with irradiated CD32-transfected fibroblasts and IL-4 upregulates AID mRNA; Figure 19A (FACS analysis) and 19B (immunofluorescent microscopy) demonstrate a parallel increase in AID protein. A series of 16 patients shows that the extent of such upregulation is highly variable (Figure 19C).

Using the dye CFSE to track CDS⁺CD19⁺ cell division in this same culture system, AID protein expression is increasingly upregulated on the most divided cells (Figure 20A); this finding is consistent in all samples where dividing cells are found although the rate and extent of AID upregulation varies (Figure 20B). Thus, the proliferative compartment is indeed the major source of AID⁺ cells.

AID activity is known to cause double strand breaks (DSBs) within DNA, for example in immunoglobulin switch regions during class switch recombination. DSBs (visualized using an antibody to phospho-histone H2A.X) are much more marked in the most divided (CD23⁺CFSE^{dim}) cells compared to no/minimally divided (CD23⁺CFSE^{bright}) cells (Figure 21).

Taken together, these data indicate that AID expression in CLL predominates in cells that are dividing or have a recently divided phenotype. AID activity may subsequently result in disease progression by inducing mutations in genes throughout the genome of a CLL cell, ""clonal evolution". We contend that this process could be abated by preferential targeting of the "proliferative compartment" in CLL.

Throughout this specification various publications are referred to in parenthesis. Full citations for these references are identified below.

### List of Cited References

1. Chiorazzi N, Rai KR, Ferrarini M. Chronic Lymphocytic Leukemia. N Engl J Med. 2005;352:804-815.
2. Almasri NM, Duque RE, Iturraspe J, Everett E, Braylan RC. Reduced expression of CD20 antigen as a characteristic marker for chronic lymphocytic leukemia. Am J Hematol. 1992;40:259-263.
3. Byrd JC, Peterson BL, Morrison VA, et al. Randomized phase 2 study of fludarabine with concurrent versus sequential treatment with rituximab in symptomatic, untreated patients with B-cell chronic lymphocytic leukemia: results from Cancer and Leukemia Group B 9712 (CALGB 9712). Blood. 2003;101:6-14.
4. Castro JE, Sandoval-Sus JD, Bole J, Rassenti L, Kipps TJ. Rituximab in combination with high-dose methylprednisolone for the treatment of fludarabine refractory high-risk chronic lymphocytic leukemia. Leukemia. 2008;22:2048-2053.
5. Sarfati M. CD23 and chronic lymphocytic leukemia. Blood Cells. 1993;19:591-596.
6. Pathan NI, Chu P, Hariharan K, Cheney C, Molina A, Byrd J. Mediation of apoptosis by and antitumor activity of lumiliximab in chronic lymphocytic leukemia cells and CD23+ lymphoma cell lines. Blood. 2008;111:1594-1602.
7. Damle RN, Wasil T, Fais F, et al. Ig V gene mutation status and CD38 expression as novel prognostic indicators in chronic lymphocytic leukemia. Blood. 1999;94:1840-1847.
8. Pascual V, Liu YJ, Magalski A, de Bouteiller O, Banchereau J, Capra JD. Analysis of somatic mutation in five B cell subsets of human tonsil. J Exp Med. 1994;180:329-339.
9. Deaglio S, Vaisitti T, Bergui L, et al. CD38 and CD100 lead a network of surface receptors relaying positive signals for B-CLL growth and survival. Blood. 2005;105:3042-3050.
10. Damle RN, Temburni S, Calissano C, et al. CD38 expression labels an activated subset within chronic lymphocytic leukemia clones enriched in proliferating B cells. Blood. 2007;110:3352-3359.
11. Scholzen T, Gerdes J. The Ki-67 protein: from the known and the unknown. J Cell Physiol. 2000;182:311-322.
12. Messmer BT, Messmer D, Allen SL, et al. In vivo measurements document the dynamic cellular kinetics of chronic lymphocytic leukemia B cells. J Clin Invest. 2005;115:755-764.
13. Alinari L, Lapalombella R, Andritsos L, Baiocchi RA, Lin TS, Byrd JC. Alemtuzumab (Campath-1H) in the treatment of chronic lymphocytic leukemia. Oncogene. 2007;26:3644-3653.
14. Burger JA, Kipps TJ. Chemokine receptors and stromal cells in the homing and homeostasis of chronic lymphocytic leukemia B cells. Leuk Lymphoma. 2002;43:461-466.
15. Burger JA, Kipps TJ. CXCR4: a key receptor in the crosstalk between tumor cells and their microenvironment. Blood. 2006;107:1761-1767.
16. Barretina J, Junca J, Llano A, et al. CXCR4 and SDF-1 expression in B-cell chronic lymphocytic leukemia and stage of the disease. Ann Hematol. 2003;82:500-505.
17. Gattei V, Bulian P, Del Principe MI, et al. Relevance of CD49d protein expression as overall survival and progressive disease prognosticator in chronic lymphocytic leukemia. Blood. 2007.
18. Rossi D, Zucchetto A, Rossi FM, et al. CD49d expression is an independent risk factor of progressive disease in early stage chronic lymphocytic leukemia. Haematologica. 2008;93:1575-1579.
19. O'Brien SM, Kantarjian HM, Cortes J, et al. Results of the fludarabine and cyclophosphamide combination regimen in chronic lymphocytic leukemia. J Clin Oncol. 2001;19:1414-1420.
20. Tam CS, O'Brien S, Wierda W, et al. Long-term results of the fludarabine, cyclophosphamide, and rituximab regimen as initial therapy of chronic lymphocytic leukemia. Blood. 2008;112:975-980.
21. Schweighofer CD, Ritgen M, Eichhorst BF, et al. Consolidation with alemtuzumab improves progression-free survival in patients with chronic lymphocytic leukaemia (CLL) in first remission: long-term follow-up of a randomized phase III trial of the German CLL Study Group (GCLLSG). Br J Haematol. 2009;144:95-98.
22. Ferrarini M, Chiorazzi N. Recent advances in the molecular biology and immunobiology of chronic lymphocytic leukemia. Semin Hematol. 2004;41:207-223.
23. Lichtman MA BE, Kipps TJ, Selighsohn U, Kaushansky K, Prchal JT Williams Hematology (ed 7th edition): McGraw-Hill Medical; 2006.
24. Caligaris-Cappio F. Biology of chronic lymphocytic leukemia. Rev Clin Exp Hematol. 2000;4:5-21.
25. Ghia P, Caligaris-Cappio F. The indispensable role of microenvironment in the natural history of low-grade B-cell neoplasms. Adv Cancer Res. 2000;79:157-173.
26. Mehta K, Shahid U, Malavasi F. Human CD38, a cell-surface protein with multiple functions. Faseb J. 1996;10:1408-1417.
27. Ferrero E, Malavasi F. Human CD38, a leukocyte receptor and ectoenzyme, is a member of a novel eukaryotic gene family of nicotinamide adenine dinucleotide+-converting enzymes: extensive structural homology with the genes for murine bone marrow stromal cell antigen 1 and aplysian ADP-ribosyl cyclase. J Immunol. 1997;159:3858-3865.
28. Durig J, Naschar M, Schmucker U, et al. CD38 expression is an important prognostic marker in chronic lymphocytic leukaemia. Leukemia. 2002;16:30-35.
29. Pepper C, Ward R, Lin TT, et al. Highly purified CD38(+) and CD38(-) sub-clones derived from the same chronic lymphocytic leukemia patient have distinct gene expression signatures despite their monoclonal origin. Leukemia. 2007;21:687-696.
30. Busch R, Neese RA, Awada M, Hayes GM, Hellerstein MK. Measurement of cell proliferation by heavy water labeling. Nat Protoc. 2007;2:3045-3057.
31. Neese RA, Misell LM, Turner S, et al. Measurement in vivo of proliferation rates of slow turnover cells by 2H2O labeling of the deoxyribose moiety of DNA. Proc Natl Acad Sci U S A. 2002;99:15345-15350.
32. Brown DC, Gatter KC. Ki67 protein: the immaculate deception? Histopathology. 2002;40:2-11.
33. Endl E, Gerdes J. The Ki-67 protein: fascinating forms and an unknown function. Exp Cell Res. 2000;257:231-237.
34. Lopez-Giral S, Quintana NE, Cabrerizo M, et al. Chemokine receptors that mediate B cell homing to secondary lymphoid tissues are highly expressed in B cell chronic lymphocytic leukemia and non-Hodgkin lymphomas with widespread nodular dissemination. J Leukoc Biol. 2004;76:462-471.
35. Cyster JG. Chemokines and cell migration in secondary lymphoid organs. Science. 1999;286:2098-2102.
36. Campbell JJ, Hedrick J, Zlotnik A, Siani MA, Thompson DA, Butcher EC. Chemokines and the arrest of lymphocytes rolling under flow conditions. Science. 1998;279:381-384.
37. Okada T, Ngo VN, Ekland EH, et al. Chemokine requirements for B cell entry to lymph nodes and Peyer's patches. J Exp Med. 2002;196:65-75.
38. Ansel KM, Ngo VN, Hyman PL, et al. A chemokine-driven positive feedback loop organizes lymphoid follicles. Nature. 2000;406:309-314.
39. Burger JA, Burger M, Kipps TJ. Chronic lymphocytic leukemia B cells express functional CXCR4 chemokine receptors that mediate spontaneous migration beneath bone marrow stromal cells. Blood. 1999;94:3658-3667.
40. Ghobrial IM, Bone ND, Stenson MJ, et al. Expression of the chemokine receptors CXCR4 and CCR7 and disease progression in B-cell chronic lymphocytic leukemia/ small lymphocytic lymphoma. Mayo Clin Proc. 2004;79:318-325.
41. Calissano C, Damle RN, Hayes G, Murphy EJ, Hellerstein MK, Moreno C, Sison C, Kaufinan MS, Kolitz JE, Allen SL, Rai KR, Chiorazzi N. In vivo intraclonal and interclonal kinetic heterogeneity in B-cell chronic lymphocytic leukemia. Blood. 2009 Nov 26;114(23):4832-42.

## Claims

1. An agent which is a bispecific antibody and which targets cell surface membrane antigens expressed preferentially on cells of the proliferative compartment of a chronic lymphocytic leukemia (CLL) clone wherein the agent binds to (i) CD23 and CD52, (ii) CD19 and CD23, (iii) CD19 and CD27, (iv) CD19 and CD38, (v) CD19 and CD48, (vi) CD19 and CD49d, (vii) CD19 and CD52, (viii) CD19 and CD62L, and (ix) CD19 and CD100; (x) CD20 and CD11a, (xi) CD20 and CD23, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD19 and CD11a, (xxv) CD23 and CD62L, or (xxvi) CD23 and CD100, for use in a method of treating CLL in an inductive regimen.

2. The agent of claim 1, for use in a method of treating CLL in an inductive regimen, wherein the agent is a bispecific antibody that binds to CD23 and CD52.

3. The agent of claim 1, for use in a method of treating CLL in an inductive regimen, wherein the agent is a bispecific antibody that binds to CD20 and CD52.

4. The agent of any one of claims 1 to 3, for use in a method of treating CLL in an inductive regimen, which is conjugated with a toxin or radioligand.

5. An agent which is a bispecific antibody that binds to (i) CD23 and CD52, (ii) CD19 and CD23, (iii) CD19 and CD27, (iv) CD19 and CD38, (v) CD19 and CD48, (vi) CD19 and CD49d, (vii) CD19 and CD52, (viii) CD19 and CD62L, and (ix) CD19 and CD100; (x) CD20 and CD11a, (xi) CD19 and CD11a, (xii) CD20 and CD27, (xiii) CD20 and CD38, (xiv) CD20 and CD48, (xv) CD20 and CD49d, (xvi) CD20 and CD52, (xvii) CD20 and CD62L, and (xviii) CD20 and CD100; (xix) CD23 and CD11a, (xx) CD23 and CD27, (xxi) CD23 and CD38, (xxii) CD23 and CD48, (xxiii) CD23 and CD49d, (xxiv) CD23 and CD62L, or (xxv) CD23 and CD100.

6. The agent of claim 5, wherein the agent is a bispecific antibody that binds to CD20 and CD52.

7. The agent of claim 5 or 6, which is conjugated with a toxin or radioligand.

8. A pharmaceutical composition comprising the agent of any one of claims 5 to 7.

## Patentansprüche

1. Ein Agens, bei dem es sich um einen bispezifischen Antikörper handelt und das Zelloberflächenmembranantigene, die präferentiell an Zellen des proliferativen Kompartiments eines CLL(chronische lymphatische Leukämie)-Klons exprimiert werden, ansteuert, wobei das Agens am (i) CD23 und CD52, (ii) CD19 und CD23, (iii) CD19 und CD27, (iv) CD19 und CD38, (v) CD19 und CD48, (vi) CD19 und CD49d, (vii) CD19 und CD52, (viii) CD19 und CD62L und (ix) CD19 und CD100; (x) CD20 und CD11a, (xi) CD20 und CD23, (xii) CD20 und CD27, (xiii) CD20 und CD38, (xiv) CD20 und CD48, (xv) CD20 und CD49d, (xvi) CD20 und CD52, (xvii) CD20 und CD62L und (xviii) CD20 und CD100; (xix) CD23 und CD11a, (xx) CD23 und CD27, (xxi) CD23 und CD38, (xxii) CD23 und CD48, (xxiii) CD23 und CD49d, (xxiv) CD19 und CD11a, (xxv) CD23 und CD62L oder (xxvi) CD23 und CD100 bindet, zur Verwendung in einem Verfahren zur Behandlung von CLL in einem induktiven Behandlungsschema.

2. Agens nach Anspruch 1 zur Verwendung in einem Verfahren zur Behandlung von CLL in einem induktiven Behandlungsschema, wobei es sich bei dem Agens um einen bispezifischen Antikörper, der an CD23 und CD52 bindet, handelt.

3. Agens nach Anspruch 1 zur Verwendung in einem Verfahren zur Behandlung von CLL in einem induktiven Behandlungsschema, wobei es sich bei dem Agens um einen bispezifischen Antikörper, der an CD20 und CD52 bindet, handelt.

4. Agens nach einem der Ansprüche 1 bis 3 zur Verwendung in einem Verfahren zur Behandlung von CLL in einem induktiven Behandlungsschema, das mit einem Toxin oder einem Radioliganden konjugiert ist.

5. Agens, bei dem es sich um einen bispezifischen Antikörper, der an (i) CD23 und CD52, (ii) CD19 und CD23, (iii) CD19 und CD27, (iv) CD19 und CD38, (v) CD19 und CD48, (vi) CD19 und CD49d, (vii) CD19 und CD52, (viii) CD19 und CD62L und (ix) CD19 und CD100; (x) CD20 und CD11a, (xi) CD19 und CD11a, (xii) CD20 und CD27, (xiii) CD20 und CD38, (xiv) CD20 und CD48, (xv) CD20 und CD49d, (xvi) CD20 und CD52, (xvii) CD20 und CD62L und (xviii) CD20 und CD100; (xix) CD23 und CD11a, (xx) CD23 und CD27, (xxi) CD23 und CD38, (xxii) CD23 und CD48, (xxiii) CD23 und CD49d, (xxiv) CD23 und CD62L oder (xxv) CD23 und CD100 bindet, handelt.

6. Agens nach Anspruch 5, wobei es sich bei dem Agens um einen bispezifischen Antikörper, der an CD20 und CD52 bindet, handelt.

7. Agens nach Anspruch 5 oder 6, das mit einem Toxin oder einem Radioliganden konjugiert ist.

8. Pharmazeutische Zusammensetzung, umfassend das Agens nach einem der Ansprüche 5 bis 7.

## Revendications

1. Agent qui est un anticorps bispécifique et qui cible des antigènes de membrane de surface cellulaire exprimés de façon préférentielle sur des cellules du compartiment prolifératif d'une leucémie lymphocytaire chronique (LLC), l'agent se liant à (i) CD23 et CD52, (ii) CD19 et CD23, (iii) CD19 et CD27, (iv) CD19 et CD38, (v) CD19 et CD48, (vi) CD19 et CD49d, (vii) CD19 et CD52, (viii) CD19 et CD62L, et (ix) CD19 et CD100 ; (x) CD20 et CD11a, (xi) CD20 et CD23, (xii) CD20 et CD27, (xiii) CD20 et CD38, (xiv) CD20 et CD48, (xv) CD20 et CD49d, (xvi) CD20 et CD52, (xvii) CD20 et CD62L, et (xviii) CD20 et CD100 ; (xix) CD23 et CD11a, (xx) CD23 et CD27, (xxi) CD23 et CD38, (xxii) CD23 et CD48, (xxiii) CD23 et CD49d, (xxiv) CD19 et CD11a, (xxv) CD23 et CD62L, ou (xxvi) CD23 et CD100, pour utilisation dans un procédé de traitement de LLC dans un régime d'induction.

2. Agent de la revendication 1, pour utilisation dans un procédé de traitement de LLC dans un régime d'induction, l'agent étant un anticorps bispécifique qui se lie à CD23 et CD52.

3. Agent de la revendication 1, pour utilisation dans un procédé de traitement de LLC dans un régime d'induction, l'agent étant un anticorps bispécifique qui se lie à CD20 et CD52.

4. Agent de l'une quelconque des revendications 1 à 3, pour utilisation dans un procédé de traitement de LLC dans un régime d'induction, qui est conjugué avec une toxine ou un radioligand.

5. Agent qui est un anticorps bispécifique qui se lie à (i) CD23 et CD52, (ii) CD19 et CD23, (iii) CD19 et CD27, (iv) CD19 et CD38, (v) CD19 et CD48, (vi) CD19 et CD49d, (vii) CD19 et CD52, (viii) CD19 et CD62L, et (ix) CD19 et CD100 ; (x) CD20 et CD11a, (xi) CD19 et CD11a, (xii) CD20 et CD27, (xiii) CD20 et CD38, (xiv) CD20 et CD48, (xv) CD20 et CD49d, (xvi) CD20 et CD52, (xvii) CD20 et CD62L, et (xviii) CD20 et CD100 ; (xix) CD23 et CD11a, (xx) CD23 et CD27, (xxi) CD23 et CD38, (xxii) CD23 et CD48, (xxiii) CD23 et CD49d, (xxiv) CD23 et CD62L, ou (xxv) CD23 et CD100.

6. Agent de la revendication 5, l'agent étant un anticorps bispécifique qui se lie à CD20 et CD52.

7. Agent de la revendication 5 ou 6, qui est conjugué avec une toxine ou un radioligand.

8. Composition pharmaceutique comprenant l'agent de l'une quelconque des revendications 5 à 7.
